# EUROPEAN PATENT SPECIFICATION

(11) **EP 4 244 861 B1**
(45) Date of publication and mention of the grant of the patent: **22.04.2026**
(21) Application number: 21830487.1
(22) Date of filing: 12.11.2021
(51) Int. Cl.: G16C 20/90, G06Q 50/00

(54) **DEVICE, SYSTEM, AND METHOD FOR VERIFICATION OF ANALYSIS DATA**
VORRICHTUNG, SYSTEM UND VERFAHREN ZUR VERIFIZIERUNG VON ANALYSEDATEN
DISPOSITIF, SYSTÈME ET PROCÉDÉ DE VÉRIFICATION DE DONNÉES D'ANALYSE

(30) Priority: 13.11.2020 US 202063113521 P
(43) Date of publication of application: 20.09.2023
(73) Proprietor: DH Technologies Development PTE. Ltd., Singapore 739256 (SG)
(72) Inventor: TATE, Stephen, Concord, L4K 4V8 (CA); BAKER, Charles Andrew Hugh, Concord, L4K 4V8 (CA)
(74) Representative: Maiwald GmbH
(86) International application number: PCT/IB2021/060525
(87) International publication number: WO 2022/101856

(56) References cited:
- US-A1- 2020 021 444
- JAQUET-CHIFFELLE DAVID-OLIVIER ET AL: "Tamperproof timestamped provenance ledger using blockchain technology", FORENSIC SCIENCE INTERNATIONAL: DIGITAL INVESTIGATION, ELSEVIER, AMSTERDAM, NL, vol. 33, 12 May 2020 (2020-05-12), XP086203778, ISSN: 2666-2817, [retrieved on 20200512], DOI: 10.1016/J.FSIDI.2020.300977

## Description

### INTRODUCTION

Analysis devices, such as mass analysis instruments are generally used to perform characterize the composition of samples, including for instance, pharmaceutical samples in drug trials, and the like. Mass spectrometry (MS) is an analytical technique for determining the elemental composition of test substances with both qualitative and quantitative applications. MS can be useful for identifying unknown compounds, determining the isotopic composition of elements in a molecule, determining the structure of a particular compound by observing its fragmentation, and quantifying the amount of a particular compound in a sample, among other things. Given its sensitivity and selectivity, MS is particularly important in life science applications. A laboratory may have one mass analysis instrument or hundreds, depending upon their needs. US 2020/021444 A1 discloses techniques for managing analytical information using distributed ledger technology.

### SUMMARY

The invention is defined in the appended claims. The present technology relates to verification of mass analysis results from a mass analysis instrument. In an aspect the invention is a method according to claim 1 for generating verified mass analysis results for a sample. The method includes acquiring process data for performing a mass analysis test on the sample; performing, by a mass analysis instrument, the mass analysis test based on the acquired process data to generate mass analysis results for the sample; and recording machine-level characteristics for the mass analysis instrument during the mass analysis test. The method also includes generating verification data that includes at least a portion of the recorded machine-level characteristics; generating a secure analytical data file that includes the verification data and at least a portion of the mass analysis results; and transmitting the secure analytical data file.

In an example, the method further includes logging at least one of calibration routine data, maintenance routine data, and cleaning routine data, wherein the verification data further includes the logged at least one of calibration routine data, maintenance routine data, and cleaning routine data. In another example, the process data is acquired from a server managed by an entity requesting the mass analysis test. In a further example, the secure analytical data file is transmitted to the server. In still another example, the method also includes encrypting the secure analytical data file prior to transmission. In yet another example, at least a portion of the secure analytical data file is stored as a block in a blockchain. According to the invention, the method further includes comparing the machine-level characteristics to the process data to determine whether the mass analysis test was performed in accordance with the process data; and based on the comparison, generating a verification certificate, wherein the verification certificate is incorporated into the verification data.

In another aspect, the invention is a mass analysis instrument according to claim 6 comprising a communication unit and a memory storing: condition data at least defining a process for analyzing the sample; a cryptographic function; and a key for input to the cryptographic function. The mass analysis instrument also includes a controller configured to: receive a sample identifier of the sample; control, according to the condition data, mass analysis instrument to analyze the sample according to mass to produce mass analysis data; perform, using at least the cryptographic function, the key, and the mass analysis data, a cryptographic operation to generate a cryptographic identifier; generate a verification file storing the cryptographic identifier in association with the sample identifier; and one or more of: store, in the memory, the verification file; and transmit, using the communication unit, the verification file for storage in a remote memory.

In an example, the condition data further includes one or more of: an analysis cycle count of the mass analysis instrument; calibration data of the mass analysis instrument; test data of the mass analysis instrument; instrument log data of the mass analysis instrument; a serial number; and a manufacturer identifier. In another example, the verification file has a blockchain format, the verification file further includes a respective cryptographic identifier of a previous verification file in a blockchain. In yet another example, the controller is further configured to generate a first block in the blockchain using one or more of: the sample identifier; calibration data of the mass analysis instrument; test data of the mass analysis instrument; instrument log data of the mass analysis instrument; a serial number; and a manufacturer identifier. In still another example, the cryptographic function includes a hash function, the key comprises a hashing key, and the cryptographic identifier comprises a combination of the mass analysis data and the sample identifier generated using the hash function and the hash key.

In another example, the controller is further configured to encrypt the verification file prior to one or more of storage and transmission. In a further example, the controller is further configured to: receive, via the communication unit, from a remote computing device, the key and process data defining the process; and store, in the memory, the key and the process data in the condition data. According to the invention, the controller is further configured to: compare the process as stored in the condition data with an implemented process performed by the mass analysis instrument; and based on the process and the implemented process matching, generate the verification file. In yet another example, the mass analysis instrument further includes a sample identifier reader, and wherein the controller is further configured to determine the sample identifier by controlling the sample identifier reader to read the sample identifier from the sample.

In another aspect, the invention is a method according to claim 12 for procuring and verifying mass analysis results. The method includes generating a request for mass analysis tests of samples by a plurality of mass analysis instruments, wherein the request includes process data for performing the mass analysis tests; transmitting the request to the plurality of mass analysis instruments; receiving, from the plurality of mass analysis instruments, secure analytical data files, wherein the secure analytical data files include mass analysis results for the samples and verificatior data, wherein: the verification data includes at least a portion of recorded machine-level characteristics for the mass analysis instrument during the mass analysis test; and the mass analysis results are verified by comparing the machine-level characteristics to the process data to determine whether the mass analysis test was performed in accordance with the process data, and based on the comparison, generating a verification certificate, wherein the verification certificate is incorporated into the verification data; and performing one or more verification operations on the received secure analytical data files.

In an example, the process data includes at least one of settings and parameters for the mass analysis instrument. In another example, the method further includes encrypting the request with a public key of one or more of the plurality of mass analysis instruments. In a further example, the one or more verification operations include decrypting the secure analytical data files with a private key. In still another example, the method also includes generating a first block in a study blockchain based on data in the request. In yet another example, the first block includes data in the request and a first hash of the data in the request. In still yet another example, at least a portion of the received secure analytical data files is provided within a second block of the study blockchain.

This Summary is provided to introduce a selection of concepts in a simplified form that are further described below in the Detailed Description. This Summary is not intended to be used to limit the scope of the claimed subject matter. Additional aspects, features, and/or advantages of examples will be set forth in part in the description which follows and, in part, will be apparent from the description, or may be learned by practice of the disclosure.

### BRIEF DESCRIPTIONS OF THE DRAWINGS

For a better understanding of the various examples described herein and to show more clearly how they may be carried into effect, reference will now be made, by way of example only, to the accompanying drawings in which:
FIG. 1 depicts a system for verification of mass analysis data, according to non-limiting examples.
FIG. 2 depicts an example mass analysis instrument, according to non-limiting examples.
FIG. 3A depicts an example method for verification of mass analysis data, according to non-limiting examples.
FIG. 3B depicts another example method for verification of mass analysis data, according to non-limiting examples.
FIG. 3C depicts another example method for verification of mass analysis data, according to non-limiting examples.
FIG. 4 depicts the system of FIG. 1 generating mass analysis data, condition data, and a device analysis identifier, according to non-limiting examples.
FIG. 5 depicts the system of FIG. 1 generating a first block of a blockchain to verify mass analysis data, according to non-limiting examples.
FIG. 6 depicts the system of FIG. 1 generating a second block of the blockchain, the second block comprising a verification file for the mass analysis data, according to non-limiting examples.
FIG. 7 depicts the system of FIG. 1 storing the blockchain, according to non-limiting examples.
FIG. 8 depicts the system of FIG. 1 generating a blockchain to verify operation of a mass analysis instrument, according to non-limiting examples.

### DETAILED DESCRIPTION

As discussed above, mass analysis instruments may be used for characterizing the composition of samples, including for instance, pharmaceutical samples in drug trials, and the like. In some fields it is useful to collect and retain sample analysis results for future including identification and verification of the analysis that was performed on the sample. For instance, verification of the analysis data may be important for approval of pharmaceuticals for use by the general population. In other cases, a drug test may be being conducted for doping assertions, and the test results are likely to be challenged at a later date.

The current practice for verifying mass analysis results is to rerun the mass-based analysis to verify that the same results are received a second time. Paper records relating to the processes used in performing the mass analysis and/or calibration data of mass analysis device may also be searched for and retrieved if available. Such methods require significant burdens, including the requirement of retaining a portion of the sample for later testing. In some examples, retaining a portion of the sample is impossible because the sample is entirely consumed during the testing process.

Among other things, the present technology helps alleviate the above-identified problem by utilizing a verification process and trust-based system to confirm the integrity of the results, secure the transfer of the data and related information, and authenticate the instrument parameters and records. The technology may operate, at least in part, on the machine-level of the mass analysis instrument, where machine-state data during the performed test may be stored, secured, and provided with the mass analysis results themselves. The mass analysis instrument may also encrypt and sign the mass analysis results along with the machine-state data to provide a secure record of the results and the test conditions. In some examples, a blockchain may be utilized to record the results and/or the machine-state data to help prevent future tampering or adjustment of the data.

As an example implementation of the present technology, a testing authority (such as the World Anti-Doping Agency (WADA)) may first generate an instruction to conduct testing of a sample. The instruction may include specific details (e.g., settings or parameters) for how the testing is to be performed by or with the mass analysis instrument. The instruction may include cryptographic information and also be incorporated or presented as a genesis block of blockchain. The sample to be tested may then be provided to testing laboratory or facility, such as a facility of a Contract Research Organization (CRO). In some examples, the CRO may include multiple satellite locations and the testing instructions may be distributed across the locations for processing of samples. The mass analysis instruments of the facilities may then perform the mass analysis tests on the sample according to the instruction to generate mass analysis results. The mass analysis results, along with the machine-state data, may be incorporated into a secured analytical data file that is transmitted back to a central CRO location or to the test-ordering entity. Generating the secured analytical data file may be based on the cryptographic information received with the instruction. For example, the secured analytical data file may be incorporated as another block in the blockchain. The CRO or test-ordering entity may then be able to verify or confirm the results in the secured analytical data file by performing a blockchain lookup to confirm the integrity and/or chain of trust of the secured analytical data file.

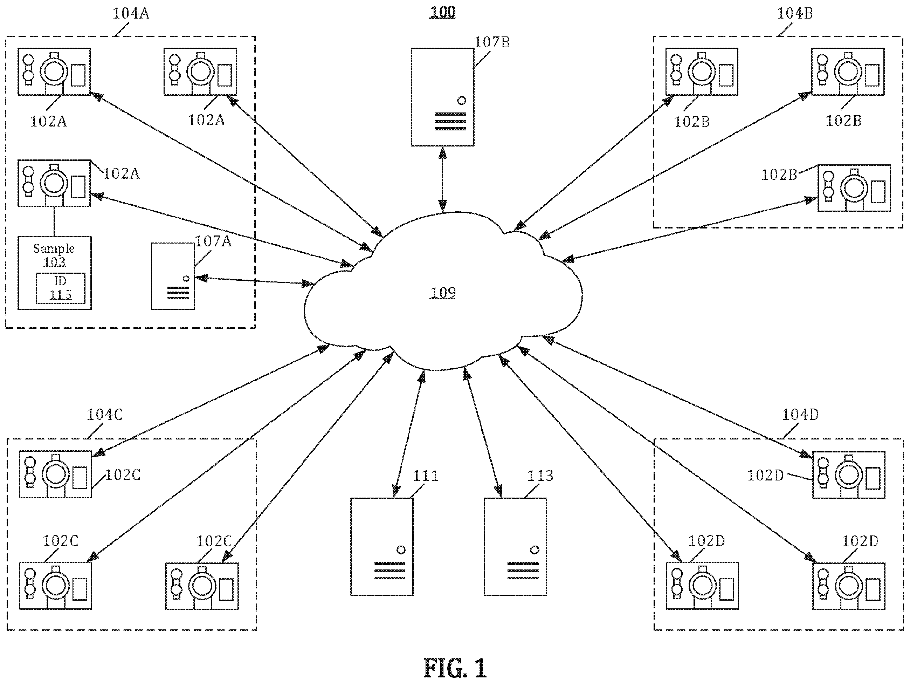

FIG. 1 depicts a system 100 for verification of mass analysis data. The system may include a plurality a mass analysis instruments 102. The mass analysis instruments 102 may be housed in a particular facility 104, such as a laboratory, university, building, campus, or similar type of facility 104. In FIG. 1, four facilities 104A-D are depicted. More specifically, system 100 includes a first facility 104A, a second facility 104B, a third facility 104C, and a fourth facility 104D. The first facility 104A includes a first plurality of mass analysis instruments 102A. The second facility 104B includes a second plurality of mass analysis instruments 102B. The third facility 104C includes a third plurality of mass analysis instruments 102C. The fourth facility 104D includes a fourth plurality of mass analysis instruments 102D.

Each of the mass analysis instruments 102 may include a mass spectrometer, a sample separator (e.g., including, but not limited to, a liquid chromatography device), and/or similar devices for analyzing the composition of an object. For instance, each of the mass analysis instruments 102 may include a stand-alone mass spectrometer, an on-line liquid chromatography-mass spectrometry (LC-MS), an on-line gas chromatography-mass spectrometry (GC-MS) system, a Fourier-transform ion cyclotron resonance mass spectrometer (FT-ICR-MS), a differential mobility spectrometer (DMS-MS), and/or a tandem mass spectrometry system (MS-MS), among other types of mass analysis systems. While only three mass analysis instruments 102 are depicted as being in each facility 104, it should be appreciated that more or fewer mass analysis instruments 102 may be housed within each facility 104. For example, some facilities 104 may house hundreds of mass analysis instruments 102.

The mass analysis instruments 102 are configured to receive a sample 103 and generate mass analysis results for the received sample. Testing of a sample generally requires multiple operations, including sample introduction, analyte ionization, mass analysis and ion detection, and data processing. Sample introduction may involve the mass analysis instrument 102 receiving an individual sample, multiple samples, and may also include chromatographic separation. During the analyte ionization operation, the sample or analyte from the sample introduction operation is ionized. For example, the mass analysis instrument 102 may produce gas phase ions that are suitable for use in the mass analysis and ion detection operations of the testing procedure. There are many different types of ionization techniques that can be used, such as electron ionization, chemical ionization, electrospray ionization, and matrix-assisted laser desorption ionization, among other techniques.

Once the ions are generated, the ions (having a mass m and z elementary charges e) are accelerated along an ion path of the mass spectrometer where additional processing steps to manipulate the ions, such as mass filtering, fragmenting, collision, etc. may take place. The resulting ions after the processing steps may be detected by a detector of the mass analysis instrument 102, so that the mass-to-charge ratio of the ion can be determined. Different types of mass analyzers or mass filters may be used to accomplish the manipulation or acceleration of the ions to allow for such types of detection. Some examples include quadrupole mass analyzers, ion-trap mass analyzers, time-of-flight mass analyzers, and orbitrap mass analyzers, among others. These analyzers and techniques generally utilize a vacuum chamber, where the low pressure is generated via a vacuum pump system that often includes at least one turbo pump operating in combination with a roughing pump.

Detection of the ions may be performed through the use of various detectors or detection systems. Some example detection systems utilize an electron multiplier detector or a microchannel plate detector. Based on the signals from the detection system, mass analysis results are generated. The mass analysis results may be in the form of mass spectra for the sample being analyzed. A mass spectrum may represent a set of ion counts for a particular amount of time. The mass analysis results may be generated in different formats or manners. For instance, the mass analysis results may be presented or stored as a total-ion chromatogram (TIC), an extracted-ion chromatogram (XIC), a base-peak chromatogram (BPC), or other types of formats.

Each of the above operations relies on the functionality of multiple components of the mass analysis instrument 102. These functions and components may need to be periodically calibrated, maintained, and/or cleaned. If the functions and components are out of calibration or improperly performing, the mass analysis results generated by the mass analysis instrument 102 may be inaccurate. Accordingly, with the present technology the calibrations, maintenance routines, and cleaning routines may be stored and provided in association with the mass analysis results to further provide confidence in the mass analysis operations performed on a particular sample along with the generated mass analysis results. Further, the settings and/or operations performed by the user of the mass analysis instrument 102 may also be recorded.

In addition, during performance of mass analysis, the operational condition of the mass analysis instrument 102 may be described or characterized by its machine-level characteristics. Accordingly, having the machine-level characteristics associated with the mass analysis results provides an indication as to whether or not the mass analysis instrument 102 is operating within specifications, which provides additional confidence in the accuracy of mass analysis results produced. The machine-level characteristics of the mass analysis instrument 102 include characteristics of operating conditions of the components of the mass analysis instrument 102. For instance, the machine-level characteristics may include parameters and/or settings of the mass analysis instrument 102. The machine-level characteristics may also include temperatures, voltages, and/or currents of certain components of the mass analysis instrument 102. Machine-level system component information may include, for instance, voltage, current, frequency, temperature, power, fluid flow rate, uptime, status, error code, polarity, and pressure as relevant for each of the system components. The machine-level characteristics may be measured, recorded, or otherwise tracked on a continuous and/or interval basis. For instance, the machine-level characteristics may be recorded every 5-10 milliseconds (ms) or on the order of seconds, minutes, or other suitable time intervals. Additionally or alternatively, a machine-level characteristic may be recorded upon a change in the respective machine-level characteristic. In the case of a commercial mass spectrometer, such as the SCIEX Triple Quad^{™} 7500 LC-MS/MS System, hundreds to thousands of machine-level characteristic variables may be tracked and/or recorded.

Each of the mass analysis instruments 102 may be in communication with a communication network 109. The communication network may be or include a local-area network (LAN), a wide area network (WAN), and/or the Internet, among other types of networks. Thus, the mass analysis instruments 102 may transmit data to other devices connected to the communication network 109. The mass analysis instruments 102 may also receive data from other devices connected to the communication network 109.

As discussed above, the mass analysis instrument 102 receives the sample 103. The sample may include a sample identifier 115. The sample identifier 115 may be provided on a label or a container of the sample 103. The sample 103 may comprise any suitable sample that may be analyzed according to mass by the mass analysis instrument 102, and the sample 103 may be provided in any suitable format. For example, the sample 103 may be a pharmaceutical sample that is undergoing testing as part of development of a drug and/or as part of a drug trial. In other examples, the sample 103 may include samples from human beings (such as saliva and the like), which may be tested for law enforcement purposes. The sample 103 may be provided as a single sample and/or the sample 103 may include a tray of a plurality of samples, which may be similar to, or different from, each other. In examples, where the sample 103 comprises a plurality of samples, each of the plurality samples may be provided with a respective sample identifier (e.g., as labels on the tray) and/or each of the plurality samples may be associated with a respective sample identifier stored in a respective order (e.g., according to a sample order in the tray). In some examples, the plurality of samples, such as part of a tray, may be provided with a container indicia, such as a bar code, associated with the container and a Laboratory Information Management System (LIMS) may store specific sample information in association with the container indicia and a specified location on the container as is known in the art. Regardless, the mass analysis instrument 102 is generally configured to generate mass analysis data for a sample and associate the mass analysis data with a sample identifier 115.

The mass analysis instrument 102 may also include a sample identifier reader, which may be provided as one or more of a barcode reader, a quick response (QR) code reader, a camera, and the like, and/or any suitable device for reading the sample identifier 115 from the identifier indicator and/or label. Indeed, the sample identifier 115 may be provided in any suitable format (e.g., a barcode, a QR code, alphanumeric text, and the like), compatible with the sample identifier reader. The sample identifier reader may capture the sample identifier 115 and provide the data from the sample identifier 115 (e.g., in any suitable format) to the mass analysis instrument 102. Alternatively, the mass analysis instrument 102 may include at least one input device which may be used to receive input indicative of a sample identifier 115, such as a manual entry of the sample identifier 115 or by transmission from a centralized LIMS for instance.

The mass analysis instrument 102 may include a suitable computing device or suitable computing components such as a processor and memory. The computing components may be housed within the mass analysis instrument 102 itself, located adjacent to the mass analysis instrument 102, or be in electronic communication with the mass analysis hardware and components of the mass analysis instrument 102. The memory may store instructions that, when executed by the processor cause the mass analysis instrument 102 to perform a set of operations, such as the operations described herein. For example, the operations may include analyzing a sample and generating mass analysis results. The operations may also include generating a verification file using a cryptographic function, such as a hash function and the like. The verification file may include data relating to machine-level characteristics, calibration routines, maintenance routines, cleaning routines, settings of the mass analysis instrument 102, and/or operations performed by and/or with the mass analysis instrument during performance of the analysis of the sample 103.

The system 100 may also include servers 107. The servers 107 may be laboratory management servers for a particular facility 104 or facilities 104. For instance, the servers 107 may store data received from the mass analysis instrument 102, such as mass analysis results and/or data. The servers 107 may also perform encryption techniques on the received data. The servers 107 include computing components such as processors and memory for processing and storing data. In the example depicted, the servers 107 include a first server 107A and a second server 107B. The servers 107 are in communication with the communications network 109. Accordingly, the servers 107 may communicate with the mass analysis instruments 102 via the communications network 109. The servers 107 may provide storage for mass analysis data, verification data, and/or other types of data (e.g., blockchain data).

The servers 107 are generally separate from and/or remote from the mass analysis instrument 102. While the servers 107 may be described as remote from the mass analysis instruments 102, the term "remote" may include the servers 107 being in communication with the mass analysis instrument 102 via a local area network (LAN), wireless local area network (WLAN) and the like. For example, the servers 107 may be operated by same entity as one or more of the mass analysis instruments 102, and the servers 107 may be located on the same or different premises as the mass analysis instruments 102. For instance, one or more of the servers 107 may be located in the same facility 104 as one or more the mass analysis instruments 102. Further, while two servers 107 are depicted, the system 100 may comprise as few as one server 107 or more than two servers 107. In other examples, the servers 107 may be absent from the system 100, with the mass analysis data and the verification data being stored at the mass analysis instruments 102 and/or transmitted to another device. As will be appreciated, in some instances the servers 107 may comprise virtual instances operating on a same computing hardware, or group of computing hardware. In some instances, the servers 107 may comprise virtual instances operating in a cloud environment and may be distributed across an available computing resource in a distributed manner.

Some servers in the system 100 may serve specific purposes. For example, in some embodiments the system 100 may include a management server 111 and a certificate server 113. Among other functions, the management server 111 may provide process data, such as instructions for carrying out the tests of the sample. For example, the management server 111 may be operated by an entity that is requesting the analysis or testing of the sample 103. The management server 111 may issue process data or an instruction with a type of tests that are to be performed on the sample 103. The process data or instruction may be sent to the mass analysis instruments 102 that are to perform the tests on the sample 103. The process data or instruction may be sent based on a manual selection to send the process data. In other examples, the instruction may be sent based on a request for the process data or instruction from a mass analysis instrument 102.

In embodiments where certificates are employed, the certificate server 113 may create and store asymmetric key pairs for encrypting or decrypting as well as signing or validating anything that depends on a public key infrastructure (PKI) or other similar infrastructures. For instance, the certificate server 113 may issue a signed certificate to a mass analysis instrument 102 and/or a facility 104 operating the mass analysis instrument 102. When the signed certificate (along with or including a public key) is presented to the entity requesting the mass analysis data (e.g., the test-ordering entity), the recipient can cryptographically confirm the certificate server's digital signature via the certificate server's public key. Additionally, the entity requesting the mass analysis data can use the certificate to confirm that signed content (e.g., the mass analysis results and/or verification data) was sent by the mass analysis instrument 102 having the corresponding private key and that the information has not been altered since it was signed. The certificate server 113 may be operated by the same entity that operates the mass analysis instruments 102, an entity that manufactures or provides the mass analysis instruments 102, an independent third party such as a regulatory body, or other trusted entity. In an example, the manufacturer may provide a signed certificate authenticating each of the mass analysis instruments 102. The mass analysis instrument 102 may then provide that certificate with the mass analysis results to provide additional trust in the mass analysis results. In other examples, the certificate server 113 may be operated by a third-party certificate authority.

In some examples, the certificate server 113 may certify the mass analysis data and/or the verification data generated from the mass analysis instruments 102. For instance, the certificate server 113 may certify and encrypt the mass analysis results and/or the verification data. The encrypted mass analysis results and/or the verification data, along with a decryption key, may then be provided to the entity that ordered the tests of the sample. For instance, the certificate server 113 may transmit the encrypted data and the decryption key (e.g., public key) to the management server 111. Thus, the use of a certificate server 113 allows for the test-ordering entity to have more trust and confidence that the received results have not been improperly altered.

Encryption of the mass analysis results and/or verification data may comprise providing for public and private information associated with the mass analysis results and/or verification data. The private information being encrypted and requiring specified cryptographic information to view while the public information being available for review. In some aspects, the encryption of the mass analysis results and/or verification data may provide for authentication of the integrity of the information, such as by providing a cryptographic hash of some or all of the information that may subsequently be verified to confirm the integrity of the information when reviewed at a later time.

The management server 111 and/or the certificate server 113 may each comprise a computing device and/or computing components remote from the mass analysis instrument 102 and hence may alternatively be referred to as remote computing devices. The management server 111 and/or the certificate server 113 may be associated with a same or different entity as the mass analysis instrument 102 and the servers 107. For example, the management server 111 and/or the certificate server 113 may be associated with a manufacturer of the mass analysis instruments 102, which may have been purchased by the entity operating the servers 107 and/or a facility 104. While depicted as separate from each other, the management server 111 and/or the certificate server 113 may be combined.

The mass analysis instruments 102 and the servers 107 may be associated with a same entity, such as a pharmaceutical company developing a new drug (e.g., the sample 103) and/or a contract research organization (CRO) to which sample testing is being outsourced by a pharmaceutical company, and the like. However, the mass analysis instruments 102 and the servers 107 may be associated with different entities. Alternatively, the mass analysis instruments 102 and the servers 107 may be associated with a legal entity, for example a law enforcement agency conducting testing of saliva using the mass analysis instruments 102 to test for presence of controlled substances. However, the mass analysis instruments 102 and the servers 107 may be associated with any type of entity conducting testing of samples using the mass analysis instruments 102.

When blockchain technology is utilized in the present technology, the blockchain may be distributed across the mass analysis instruments 102, the servers 107, the management server 111, and/or the certificate server 113. Accordingly, multiple devices may store a copy of the blockchain, which adds additional security and verification measures for data stored in the blockchain. Multiple devices may then verify the data within the blockchain using blockchain verification techniques, for example and/or by regenerating a blockchain when access to the data used to generate the blockchain is available. As discussed further below, a blockchain may be utilized in a manner similar to a smart contract, and each device involved may generate additional blocks to add to the blockchain. For instance, the management server 111 may create the originating or genesis block of the blockchain that includes an instruction to perform the tests on the sample. The mass analysis instrument 102 performing the tests may then generate another block with the mass analysis results and/or the verification data. A blockchain for the mass analysis instrument 102 may also be generated that tracks the usage and results generated by the mass analysis instrument 102. Conveniently, portions of the blockchain may be reviewed, such as the mass analysis results, while other portions may reside with related entities, such as a manufacturer of the mass analysis instrument 102. In these embodiments, the portions of the blockchain maintained by the related entities may be called upon as may be required, for instance to verify a mass analysis instrument 102 was operating within specification when a set of mass analysis results were generated.

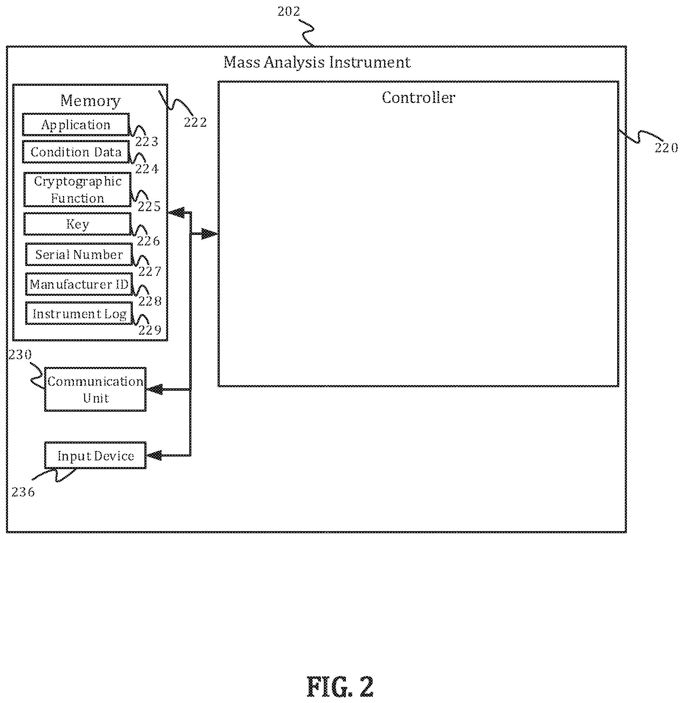

FIG. 2 depicts a schematic diagram of some components of a mass analysis instrument 202. The mass analysis instrument 202, may include or be connected to any suitable computing device or computing components, including but not limited to a circuit board, a personal computer (PC), a laptop computer, a server, and the like, and which may depend on the computing components being in a same or different housing of the mass analysis instrument 202. The mass analysis instrument 202 may include a controller 220. The mass analysis instrument 202 may also include a memory 222 storing: an application 223, instruction or condition data 224 at least defining a process for analyzing the sample 103, a cryptographic function 225, a key 226 for input to the cryptographic function 225, a device serial number 227 (e.g., a serial number of the mass analysis instrument 202), a manufacturer identifier 228 (e.g., a name of a manufacturer of the mass analysis instrument 202), and/or instrument log data 229 of the mass analysis instrument 202 (e.g., machine-level characteristics and/or a log of maintenance, calibration, and/or cleaning routines). The mass analysis instrument 202 may also include a communication unit 230 and an input device 236. The controller 220 is generally in communication with the other components using a computer bus and the like (e.g., with connections between the components of the mass analysis instrument 202 depicted as double-ended arrows). While the mass analysis instrument 202 is described with respect to including certain components, the mass analysis instrument 202 may include other components, such as a display screen, a speaker, a notification device, and the like.

The controller 220 may include one or more logic circuits, one or more processors, one or more microprocessors, one or more digital signal processors, one or more applications processors, one or more ASIC (application-specific integrated circuits) and one or more FPGA (field-programmable gate arrays), and/or another electronic device, adapted for the functionality of the controller 220. Indeed, in some examples, the controller 220 is not a generic controller and/or a generic device, but a controller and/or a device specifically configured to implement functionality for mass analysis and verification of mass analysis data. For example, in some embodiments, the controller 220 specifically includes a computer executable engine configured to implement functionality for mass analysis and verification of mass analysis data.

The memory 222 may comprise a non-volatile storage unit (e.g., Erasable Electronic Programmable Read Only Memory ("EEPROM"), Flash Memory) and a volatile storage unit (e.g., random-access memory ("RAM")). Programming instructions that implement the functional teachings of the mass analysis instrument 202 as described herein are typically maintained, persistently, in the memory 222 and used by the controller 220 which makes appropriate utilization of volatile storage during the execution of such programming instructions. Those skilled in the art recognize that the memory 222 is an example of computer readable media that can store programming instructions executable on the controller 220. Furthermore, the memory 222 is also an example of a memory unit and/or memory module and/or a non-volatile memory.

In particular, the memory 222 stores the application 223 that, when processed or executed by the controller 220, enables the controller 220 and/or the mass analysis instrument 102 to perform operations described herein. For instance, the operations may include, canning and identifying a sample identifier 115 of the sample 103; controlling, according to the condition data 224, the mass analysis instrument 202 to analyze the sample 103 generate mass analysis data; performing, using at least the cryptographic function 225, the key 226, the mass analysis data and the sample identifier 115, a cryptographic operation to generate a cryptographic identifier; generating a verification file storing the cryptographic identifier in association with the sample identifier 115; storing, in the memory 222, the verification file; and/or transmitting, using the communication unit 230, the verification file for storage in a remote memory.

Indeed, the controller 220 and/or the mass analysis instrument 202 may be operated in different modes and the application 223 may hence include a plurality of application modules or subsets of programming, with a different application module selected to be processed by the controller 220 depending a selected mode of operation. A mode of operation may be selected using the input device 236.

The communication unit 230 includes any suitable combination of wired or wireless communication units and/or communication interfaces configured to communicate with the servers 107, 111, 113 and the network 109 and the sample identifier reader, when present. The communication unit 230 may communicate in a wired and/or wireless manner as desired including, but not limited using cables, WiFi communication links, Bluetooth^{™} communication links, personal area networks, local area networks, and the like.

The optional input device 236 may comprise any suitable combination of a keyboard, pointing device, touchscreen, buttons and the like, for receiving input. For instance input indicative of the sample identifier 115 may be input via the input device 236. In some examples, the input device 236 may include a camera or scanner to scan or read the sample identifier. For example, the sample identifier 115 may be a one-dimensional or a two-dimensional barcode (e.g., a Quick Response (QR) code). The sample identifier may then be scanned or read using the camera or scanner. Once the sample identifier has been scanned or read, the mass analysis instrument 202 may extract the data from the sample identifier 115, which may include information about the sample 103 itself (e.g., sample name or composition). In some examples, the sample identifier 115 may include process data, condition data 224, and/or types of tests to be performed on the sample 103. For instance, a QR code may encode about 3 kilobytes of data. Accordingly, the QR code may include a substantial amount of information. Additionally or alternatively, the sample identifier 115 may include secure request data that can be transmitted to a management server 111 to request condition data 224, process data, or instructions for performing the tests of the sample 103.

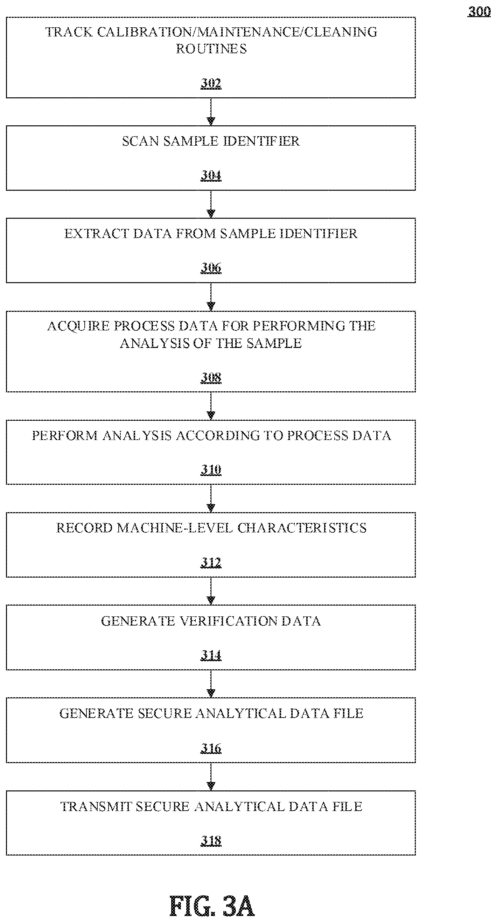

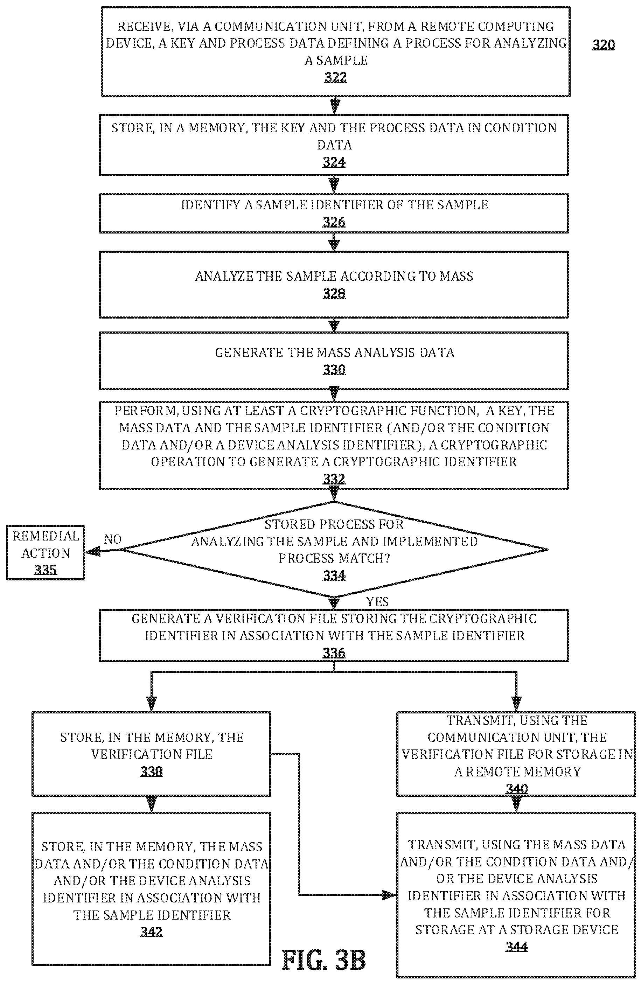

FIG. 3A depicts an example method 300 for generating verified mass analysis results from a mass analysis instrument. In order to assist in the explanation of the method 300 and the method 320 discussed further below with respect to FIG. 3B, it will be assumed that the methods 300, 320 are performed using the system 100, and at least partially by the controller 220 of the mass analysis instrument 102, for example when the controller 220 processes the application 223. Indeed, the methods 300, 320 are ways in which the system 100 and/or the mass analysis instrument 102 can be configured. Furthermore, the following discussion of the methods 300, 320 will lead to a further understanding of the system 100 and/or the mass analysis instrument 102 and their various components. However, it is to be understood that the system 100 and/or the device mass analysis instrument 102 and/or the methods 300, 320 can be varied, and need not work exactly as discussed herein in conjunction with each other, and that such variations are within the scope of present examples.

Regardless, it is to be emphasized, that the methods 300, 320 need not be performed in the exact sequence as shown, unless otherwise indicated; and likewise, various operations in the blocks of FIGS. 3A-3C may be performed in parallel rather than in sequence. It is also to be understood, however, that the methods 300, 320 can be implemented on variations of the system 100 and/or the mass analysis instrument 102 as well. Furthermore, while mass analysis instrument 102 is described as implementing and/or performing at least a portion of the operations of the methods 300, 320, it is appreciated that such operations of the methods 300, 320 may occur using the controller 220 processing application 223.

At operation 302 of method 300, calibration, maintenance, and/or cleaning routines for the mass analysis instrument 102 are tracked and logged. For example, the mass analysis instrument 102 may capture calibration data including a date and/or time when the calibration took place. The calibration state corresponding to that date and/or time may then be logged. The calibration state may include detailed calibration data about the calibration procedure that occurred. For example, the calibration state may include mass analysis results (e.g., mass spectra) corresponding to a calibration operation performed on the mass analysis instrument 102 at that date and/or time. For instance, the mass analysis results may be for a calibrant analyzed by the mass analysis instrument 102 during the calibration process. In other examples, the calibration state may comprise a simplified calibration state, such as a binary representation indicative of calibrated or not calibrated.

The manufacturer of the mass analysis instrument 102 may verify the calibration state based on the calibration data. For example, the calibration data may be sent to the manufacturer, and the manufacturer may confirm that the calibration has been completed. The verification that the calibration has been properly completed may also be stored in a log event for the calibration procedure. The verification from the manufacturer may be issued with or in the form of a digitally signed certificate. Similarly, maintenance routines and/or cleaning routines may be logged to show when the mass analysis instrument 102 was maintained and/or cleaned. In addition, the log data may include a log of tests or operations performed by the mass analysis instrument 102 since the logged calibration, maintenance, and/or cleaning event. For example, a cleaning event may result in the mass analysis instrument 102 being clean for a certain number of tests before the mass analysis instrument 102 becomes contaminated. The logged subsequent test data may also include the types of tests performed. Thus, an entity can better determine the state of the mass analysis instrument 102 at the time a particular test of a sample 103 was performed.

The calibration, maintenance, and/or cleaning routines may be stored in an encrypted manner to help prevent tampering. The encrypted calibration data may also be sent to a CRO or test-requesting entity such that the entity also has a duplicative log of the calibration, maintenance, and/or cleaning routines. For instance, the CRO and/or test-requesting entity may provide a public encryption key to the mass analysis instrument 102, and the mass analysis instrument 102 may use that public encryption key to encrypt the calibration data. Additionally or alternatively, the mass analysis instrument 102 may use a private key to digitally sign and/or encrypt the data and provide the corresponding public decryption key to the CRO and/or test-requesting entity. In other examples, symmetric encryption/decryption keys may also be utilized.

In some examples, the log data of calibration, maintenance, and/or cleaning routines may be stored as a blockchain to further prevent tampering or alteration. For example, the first calibration data logged may be hashed and stored as a genesis block for a calibration blockchain. The second calibration data (e.g., data for a calibration performed at a time after the first) and the hash from the genesis block may then be used to create a second hash for a second block in the calibration blockchain. The calibration blockchain may be distributed across multiple devices, including multiple mass analysis instruments 102, servers 107, CROs, and/or test-requesting entities. In such examples, a calibration blockchain may exist for each mass analysis instrument 102 or for a collections of mass analysis instruments 102, such as a set of mass analysis instruments 102 within a particular facility or laboratory. The blocks containing calibration data may also be stored in a larger blockchain for a particular study.

At operation 304, a sample identifier 115 is scanned by the mass analysis instrument 102. The sample identifier 115 may be scanned using a camera or scanner of the mass analysis instrument 102, as discussed above. At operation 306, data is extracted from the scanned sample identifier 115. For example, the sample identifier 115 may encode data, such as a unique identifier for the sample 103, which may be a numerical, alphabetical, or alphanumerical (e.g., hexadecimal) string that uniquely identifies the sample 103. The data from sample identifier 115 may also include process data for how the test is to be performed.

At operation 308, the process data for performing the analysis of the sample is acquired. The process data may include conditions that must be present for carrying out the test, such as specific settings or parameters for operating the mass analysis instrument 102 during performance of the test, such as calibrated tolerances and settings for controlling electronic and/or magnetic components of the mass analysis instrument 102 (e.g., magnetic quadrupoles and the like, though any suitable electronic and/or magnetic components of the mass analysis instrument 102 are within the scope of the present specification). Accordingly, the process data may include conditions that can be determined from the machine-level characteristics of the mass analysis instrument 102 when the mass analysis instrument 102 is performing the test. In some examples, the process data may not be encoded in the sample identifier 115, but the sample identifier 115 may include instructions for how to obtain the process data, such as a location where the process data is stored and/or an address for which to send a request for the process data. The mass analysis instrument 102 may then retrieve or access the process data accordingly.

At operation 310, the mass analysis tests of the sample 103 are performed according to the process data to generate mass analysis results for the sample 103. At operation 312, machine-level characteristics are recorded or stored of the mass analysis tests performed on the sample at operation 310. The machine-level characteristics may include settings or operations of the mass analysis instrument 102 that were performed, along with other types of machine-level characteristics discussed above. The particular machine-level characteristics that are recorded may be based on the process data. For instance, if the process data requires certain types of settings, at least those same settings may be recorded in the machine-level characteristics while the mass analysis test is being performed.

At operation 314, verification data is generated for the mass analysis results. The verification data may include logged calibration, maintenance, and/or cleaning data that was logged in operation 302. The verification data may also include the machine-level characteristics recorded at operation 312. Accordingly, the verification data may be utilized to determine that the mass analysis instrument 102 was in a calibrated, maintained, and/or cleaned state when the mass analysis tests were performed. The verification data may also be used to confirm that the process data was followed by comparing the process data to the machine-level characteristics in the verification data.

In some examples, the mass analysis instrument 102 (and/or a connected server 107, 111, 113) may automatically perform a comparison of the process data and the machine-level characteristics and/or calibration data to determine whether the test was performed in conformance with the process data. The test may be considered to have been performed in conformance when the machine-level characteristics match, or are within a threshold (e.g., 1%, 5%, 10%, etc.) of, the corresponding settings or parameters in the process data. A verification certificate or verification report with the results of such a comparison may also be generated and included in the verification data. In other examples, the machine-level characteristics may be transmitted to another device or third-party service to perform a validation that the process data was properly adhered to based on the machine-level characteristics. That service may then reply with a verification certificate that may be incorporated into the verification data.

At operation 316, a secure analytical data file is generated for the mass analysis results. The secure analytical data file includes the mass analysis results generated in operation 310, an identifier for the sample (e.g., from the sample identifier 115), and the verification data generated in operation 314. The secure analytical data file may be secured via encryption of all or a portion of the secure analytical data file. The secure analytical data file may be encrypted using a private key of the mass analysis instrument 102, such as a private key issued by the manufacturer and/or a certificate authority. Additionally or alternatively, the secure analytical data file may be encrypted using a public key received from the test-requesting entity, CRO, or other entity to which the results are to be provided. A cryptographic element for the secure analytical data file may be generated, such as a cryptographic hash, blockchain entry, or other element that represents the contents of the data in the secure analytical data file.

In some examples, subsets of the data in the secure analytical data file may be encrypted differently for different audiences to be able to view or decrypt the data. For instance, the calibration data may be encrypted with a first encryption method (e.g., a first public-private key pair) to allow for a wider audience, such as academics, to access the calibration data of the mass analysis instrument 102. The decryption key for the first encryption method may then be provided to that wider audience. In some examples, the calibration data or other data is not encrypted and is made publicly available, such as via a distributed blockchain. The data that is provided to a wider audience, such as the calibration data, may also be anonymized such that the particular mass analysis instrument 102 may not be identified from the data.

The machine-level characteristics may be encrypted with a second encryption method and the mass analysis results may be encrypted with a third encryption method. Thus, the machine-level characteristics may be accessed by a different audience than the actual mass analysis results, which may include more sensitive data. The cryptographic element (e.g., a hash) for each subset of data may be incorporated as data for a block in a blockchain. In such examples, the hash for that block may be generated from the hashes of the subsets of the data in the secure analytical data file and the hash from the prior block (along with a nonce value if desired).

At operation 318, the secure analytical data file is transmitted. The secure analytical data file may be transmitted as its cryptographic element(s). For instance, the secure analytical data file may be provided in its encrypted form, as a hash, and/or as part of a blockchain for the study being conducted. The secure analytical data file is transmitted to another entity, such as a CRO, the test-requesting entity, or another entity. For example, the secure analytical data file may be transmitted from the mass analysis instrument 102 to the management server 111. Because the receiving entity receives not only the mass analysis results but also the verification data, the receiving entity can have more confidence that the test was performed properly and accurately. In addition, because the receiving entity has a copy of the verification data, additional searching of logs for later verification may be no longer than necessary as the verification data is packaged with the mass analysis results. Furthermore, because the receiving entity has a copy of the verification data, any changes made to other copies will not change the copy received by the receiving entity, which results in yet another indicator of veracity.

The receiving entity may also perform additional verification techniques and augment the secure analytical data file before transmitting to another entity. For instance, a central CRO office may decrypt the machine-level characteristics and calibration log data to compare that data to the process data in order to determine that the process data was properly followed. The central CRO office may then augment the secure analytical data file with a verification certificate that certifies the process data was followed. The augmented secure analytical data file may then be transmitted to the test-requesting entity. While described as a central CRO office, other entities could also perform such verification, such as the manufacturer of the mass analysis instrument 102 or other third-party entities. In examples where blockchain is utilized, the verification certificate may also be added as a block in a blockchain.

FIG. 3B depicts another example method 320 for generating verified mass analysis results from a mass analysis instrument 102. At operation 322, the controller 220 receives, via the communication unit 230, from a remote computing device, the key 226 and process data defining a process for analyzing the sample 103 according to mass to produce mass analysis data. At operation 324, the controller 220 stores, in the memory 222, the key 226 and the process data. The process data may be stored in the condition data 224 and/or used to generate the condition data 224. The process data may hence comprise instructions which may be implemented by the controller 220 to control the mass analysis instrument 102 to analyze the sample 103 including, but not limited to, settings for controlling electronic and/or magnetic components of the mass analysis instrument 102, such as magnetic quadrupoles and the like (though any suitable electronic and/or magnetic components of the mass analysis instrument 102 are within the scope of the present specification). The condition data 224 may further comprise one or more of: an analysis cycle count of the mass analysis instrument 102; calibration data of the mass analysis instrument 102; test data of the mass analysis instrument 102; instrument log data of the mass analysis instrument 102 (e.g., the instrument log data 229); a serial number (e.g., the serial number 227); a manufacturer identifier (e.g., the manufacturer identifier 228). Hence, while the process data may be stored in the condition data 224, at least a portion of the condition data 224 may be preconfigured at the memory 222 in a factory setting and/or maintained by the controller 220 on an on-going basis as the mass analysis instrument 102 is periodically tested and/or calibrated, and/or operated.

Alternatively or additionally, however, the process data and/or the key 226 may be locally preconfigured at the memory 222, for example in a provisioning process and/or in a factory setting using the input device 236 and/or a local connection to the mass analysis instrument 102. Hence, at least the operation 322 may be optional and/or performed prior to implementation of the method 320.

At operation 326, the controller 220 identifies or detects the sample identifier 115 of the sample 103. For example, the sample identifier 115 may be received as input at the input device 236 and/or the sample identifier 115 may be determined by the controller 220 controlling the sample identifier reader to read the sample identifier 115 from an identifier indicator as described above.

At operation 328, the controller 220 controls, according to the condition data 224, the mass analysis instrument 102 to analyze the sample 103 according to mass to produce mass analysis data. For example, the controller 220 controls the mass analysis instrument 102 to operate according to the process data described with respect to the operation 322. The operation 328 may further comprise the controller 220 monitoring the mass analysis instrument 102 to determine whether the mass analysis instrument 102 is implementing the process as stored in the condition data 224 (e.g., as the process data). For example, the controller 220 may control magnetic quadrupoles, and the like, at the mass analysis instrument 102 to a target voltage, and the like, and monitor mass analysis instrument 102 to determine whether the magnetic quadrupoles achieved the target voltage. In this manner, the controller 220 may control an implemented process.

At operation 330, the mass analysis instrument 102 generates mass analysis data for the sample. The mass analysis data may comprise mass spectrometer spectra, and the like. For example, the mass analysis data may include data indicative of intensity versus mass, and the like. However, the mass analysis data may be in any suitable format including, but not limited to, an alphanumeric format.

At operation 332, the controller 220 performs, using at least the cryptographic function 225, the key 226, the mass analysis data and the sample identifier 115, a cryptographic operation to generate a cryptographic identifier. For example, the cryptographic function 225 may comprise a hash function (including, but not limited to a Secure Hash Algorithm (SHA), and the like), the key 226 may comprise a hashing key, and the cryptographic identifier may comprise a combination of the mass analysis data and the sample identifier 115 generated using the hash function and the hash key (e.g., as input to the hash function). For example, the mass analysis data and the sample identifier 115 may be combined in any suitable manner prior to hashing (e.g., the sample identifier 115 may be appended to the mass analysis data in alphanumeric format, and the like).

In some examples, the controller 220 performs, using at least the cryptographic function 225, the key 226, the mass analysis data and the condition data 224, the cryptographic operation to generate a cryptographic identifier. In these examples, the cryptographic identifier comprises a combination of the mass analysis data and the condition data 224 generated using the hash function and the hash key (e.g., as input to the hash function). For example, the mass analysis data and the condition data 224 may be combined in any suitable manner prior to hashing (e.g., the condition data 224 may be appended to the mass analysis data, and the like).

In yet further examples, the controller 220 performs, using at least the cryptographic function 225, the key 226, the mass analysis data, and a device analysis identifier, the cryptographic function 225 to generate a cryptographic identifier. A device analysis identifier may include, but is not limited to, the serial number 227, an analysis cycle identifier, and the like. For example, the controller 220 may maintain a running count of sample analysis runs and/or cycles, and the analysis cycle identifier may comprise a run number and/or a cycle number for the analysis of the sample 103 (e.g., at operation 328) in the running count of sample analysis runs and/or cycles. In some examples, the device analysis identifier may include a combination of two or more of: the sample identifier 115, the serial number 227, the analysis cycle identifier, the condition data 224, and the like. Regardless, in these examples, the mass analysis data and the device analysis identifier may be combined in any suitable manner prior to hashing (e.g., the device analysis identifier may be appended to the mass analysis data, and the like).

Indeed, in some examples, at the operation 332, the cryptographic identifier may comprise a combination of the mass analysis data and one or more of: the sample identifier 115, the condition data 224, and a device analysis identifier. In particular, the hash key, the mass analysis data and one or more of the sample identifier 115, the condition data 224, and a device analysis identifier may be used as input to the hash function to generate a hash of a combination of the mass analysis data and one or more of: the sample identifier 115, the condition data 224, and a device analysis identifier. However, while the operation 332 is described with respect to the cryptographic function 225 being a hash function, the cryptographic function may comprise any suitable cryptographic function for encrypting a combination of the mass analysis data and one or more of: the sample identifier 115, the condition data 224, and a device analysis identifier.

At operation 334, the controller 220 compares the process as stored in the condition data 224 with the implemented process performed by the mass analysis instrument 102 to determine whether the process and the implemented process match. A match may include determining that components of the mass analysis instrument 102 were controlled to the settings in the process data and/or as stored in in the condition data 224. Furthermore, a match may include matching within a tolerance range of the settings, for example voltage in the settings in the process data were implemented to within 5% at the mass analysis instrument 102. Such tolerance ranges may be predetermined, for example by drug trial regulations, and/or legal regulations, and the like. Furthermore, a record of the degree of matching may be stored in the memory 222 and/or one or more of the memories of the mass analysis instrument 102 and/or the servers 107 for use in a drug trial and/or legal proceedings.

When the process detailed by the process data and the implemented process do not match (e.g., a "NO" decision at the operation 334), the controller 220 may not generate a verification file (as described below), but rather may implement a remedial action at operation 335 (and/or continue to a next sample at the operation 326). Such a remedial action may include transmitting a notification and/or an alert to a device of an operator and/or administrator of the mass analysis instrument 102 and/or controlling a notification device (such as a display screen, a speaker and the like) of the mass analysis instrument 102 to provide a notification and/or an alert of the process and the implemented process not matching. Indeed, such a lack of a match may indicate that the mass analysis instrument 102 is in need of maintenance and/or calibration.

However, when the process and the implemented process match (e.g., a "YES" decision at operation 334), the controller 220 generates a verification file at operation 336, as described hereafter. While the operation 334 is described as being implemented after the operation 332, the operation 334 may be implemented any time after or during the operation 330, and before one or more of the operations 332, 336.

At operation 336, the controller 220 generates a verification file storing the cryptographic identifier in association with the sample identifier 115. In some examples, the verification file may comprise the sample identifier 115 stored in association with an encryption of a combination of the mass analysis data and one or more of: the sample identifier 115, the condition data 224, and a device analysis identifier.

In other examples, the verification file has a blockchain format. In such examples, the verification file includes a respective cryptographic identifier (e.g., a hash) of a previous verification file in a blockchain, as well as a copy of the data used to generate the cryptographic identifier (e.g., the combination of the mass analysis data and one or more of: the sample identifier 115, the condition data 224, and a device analysis identifier). For example, the controller 220 may be further configured to generate a first block in the blockchain (e.g., a genesis block) using one or more of: the sample identifier 115; calibration data of the mass analysis instrument 102; test data of the mass analysis instrument 102; instrument log data of the mass analysis instrument 102; the serial number 227 of the mass analysis instrument 102; and the manufacturer identifier 228 of the mass analysis instrument 102. Hence, the first block in the blockchain may comprise such data and a hash of such data. In examples where the verification file generated at the operation 336 is the second block in the blockchain, the verification file generated at the operation 336 further comprises a hash of the data of the first block.

In alternate embodiments, the genesis block may be generated by a third party, such as a regulatory entity, a contracting entity and/or the testing entity, and the controller 220 may receive the blockchain including the genesis block to add a subsequent block using the one or more of: the sample identifier 115; calibration data of the mass analysis instrument 102; test data of the mass analysis instrument 102; instrument log data of the mass analysis instrument 102; the serial number 227 of the mass analysis instrument 102; and the manufacturer identifier 228 of the mass analysis instrument 102. For simplicity the present description describes the embodiment where the blockchain originates with the controller 220 and describes the controller 220 as generating a "first block", however in the alternate embodiments the present description applies except the controller 220 is generating a subsequent block in the blockchain in response to receiving a blockchain initiated by a third party entity.

In some examples, the blocks in the blockchain may not comprise the actual data used to generate the hashes. Rather, the blocks in the blockchain may include a network address, and the like, of the data, used to generate the cryptographic identifier, as stored at a storage device, such as one or more of the memories of the mass analysis instrument 102 and/or servers 107.

For example the first block in the blockchain may comprise a hash of the sample identifier 115, calibration data of the mass analysis instrument 102, test data of the mass analysis instrument 102, instrument log data of the mass analysis instrument 102, the serial number 227, and/or the manufacturer identifier 228. However, the first block may further comprise unhashed and/or unencrypted data which enables a device analyzing the blockchain to associate the blockchain with the sample identifier 115 and/or the mass analysis instrument 102 including one or more of the serial numbers 227. Similarly, the second block in the blockchain (e.g., the verification file ) may comprise the cryptographic identifier stored in association with the sample identifier 115 and the hash of the first block, as well as a network address, and the like, of the data, used to generate the cryptographic identifier, as stored at a storage device.

Furthermore, when the method 320 is being implemented for a plurality of samples 103, such that previous blocks in the blockchain have been generated using previous implementations of the method 320, when the verification file has a blockchain format, the verification file further comprises a respective cryptographic identifier (e.g., a hash) of a previous verification file in a blockchain. Hence, the method 320 may include building a blockchain to track and verify generation of mass analysis data of a plurality of samples using the mass analysis instrument 102.

Regardless of format of the verification file, the controller 220 may, at operation 338, store, in the memory 222, the verification file; and, at operation 340, transmit, using the communication unit 230, the verification file for storage in a remote memory (such as one or more of the memories of management server 111 or servers 107). In some examples, the controller 220 may be further configured to encrypt the verification file prior to one or more of storage and transmission, for example using another key (e.g., a private key).

As depicted, the controller 220 may be further configured to, at operation 322, store in the memory 222, the mass analysis data and the condition data 224 in association with the sample identifier 115. At operation 324, the controller 220 may transmit, using the communication unit 230, the mass analysis data and the condition data 224 in association with the sample identifier 115 for storage at a storage device (which may be the same or different as the remote memory to which the verification file was transmitted at the operation 340).

Indeed, when the verification file has a blockchain format, one or more of operations 322, 324 may occur prior to generation of the verification file such that a network address, and the like, of the data, used to generate the cryptographic identifier, as stored at a storage device, is determined prior to generating the verification file. However, in other examples, the network address of the data at the storage device may be allocated prior to generating the verification file and prior to storing the data.

In some examples, blockchains described herein may include a nonce such that the blockchains may be verified using blockchain mining techniques, and the like. However, in other examples, blockchains described herein may not include a nonce. For example, the blockchains, and associated data, described herein may be stored in secure environments, for example in a pharmaceutical entity storage facility, and the like, and blockchain mining may or may not be used to verify the data associated with the blockchain.

A device having access to the verification file and the associated data (e.g., the mass analysis data), as well as the cryptographic function 225 and the key 226 may use the associated data and the verification file to verify one or more: the mass analysis data and one or more of the sample identifier 115, the condition data 224 (such as the process used to generate the mass analysis data), and the device analysis identifier, as well as to verify the integrity of a blockchain of which the verification file is a component. For example, a computing device provided with the mass analysis data (e.g., via a network address of the stored mass analysis data), the cryptographic function 225 and the key 226 and the verification file may reproduce the verification file from the mass analysis data (and, for example, sample identifiers 115 stored in the provided verification file) to verify the provided mass analysis data.

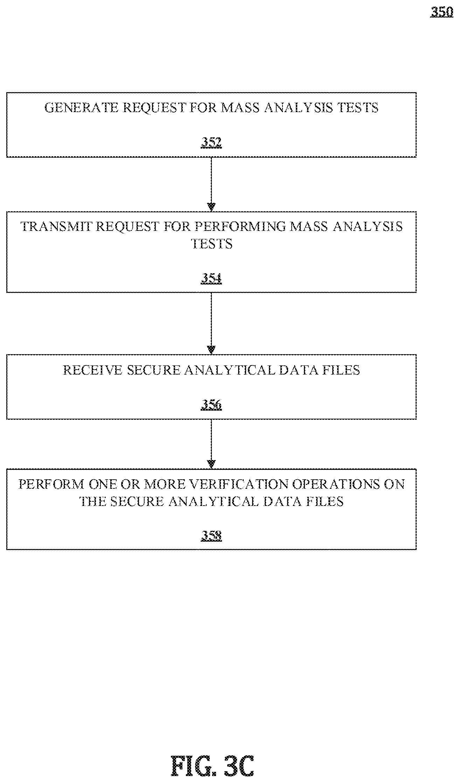

FIG. 3C depicts an example method 350 for procuring and verifying mass analysis results. The operations of method 350 may be performed by a computing device of a test-requesting entity and/or a CRO. For example, the method 350 may be performed by management server 111 or components thereof.

At operation 352, a request, having request data, for mass analysis tests of a sample or samples is generated. The request data for the mass analysis results may include process data that includes settings, parameters, and/or instructions for performing the mass analysis tests. The request data may also include the unique identifiers for the samples that are to be tested.

At operation 354, the generated request is transmitted to one or more mass analysis instruments 102. The request may be transmitted in response to a communication from one or more of the mass analysis instruments 102, such as subsequent to the mass analysis instrument scanning a sample identifier 115, or the request may be initiated based on manual input. In some examples, the request may first be encrypted prior to the request being transmitted. For instance, the request may be encrypted with a public key associated with one or more of the mass analysis instruments 102. In such an example, the mass analysis instrument 102 would then be able to decrypt the request with the corresponding private key. The request data may also be encoded into the sample identifier 115.

In some examples, the request may also be used to generate a first or genesis block of a blockchain associated with the study of the sample that is being conducted. In some examples, the request may comprise a blockchain and recipients may act to add blocks to that blockchain and/or to generate their own respective blockchains detailing operations performed by the recipient entity.

For example, the data for the first block responsive to the request may include the process data in the request. The data for the first block may also include unique identifiers for the samples that are to be tested. Thus, the first block sets forth the testing standards that are to be performed by each of the mass analysis instruments 102 such that the more consistent results may be achieved across the various mass analysis instruments 102. The first block may also identify all the samples that have been distributed for testing to different facilities. Therefore, a secured record is also created of how many samples were tested so that samples may not be later covertly omitted from the study. The request data for the block is then hashed to generate a first hash. The request data and the first hash are then stored as the first block of a study blockchain.

The mass analysis instrument 102 that receives a sample and the request then perform the requested tests according to the process data in the request to generate mass analysis results. Those mass analysis results may be combined with verification data to form a secure analytical data file, as discussed above. The secure analytical data files are received at operation 356. The secure analytical data files, or portions thereof, may have been encrypted using on or more encryption techniques to allow for authentication and/or verification, as discussed above.

At operation 358, one or more verification operations may be performed on the received secure analytical data files. For example, the verification operations may include decrypting the secure analytical data files with one or more decryption keys, which may be public keys associated with the mass analysis instruments 102 and/or private keys of the test-requesting entity depending on how the secure analytical data files were encrypted. If the decryption operation with the proper key does not result in plaintext data or properly decrypted data, the secure analytical data files may have been comprised between the time the secure analytical data files were encrypted and attempted to be decrypted.

In other examples, each of the secure analytical data files, or portions thereof, may form additional blocks in the study blockchain. The ordering of the blocks may be based on order in which the secure analytical data files were generated. For example, the first secure analytical data file generated may form the second block in the study blockchain. For instance, the data in the first generated secure analytical data file may be combined with the first hash from the first block. The combined data and first hash may then be hashed to form a second hash that is stored with the secure analytical data file in the second block. The secure analytical data file, or a portion thereof, stored in the second block may be encrypted prior to being incorporated into the block. In examples that utilize blockchain, the verification operation performed at operation 358 may also include attempts to recreate the hashes in the respective blocks. For instance, the test-requesting entity should be able to recreate the second hash of the second block by hashing the data of the second block and hash of the first block together. If that recreated hash is does not match the hash of the second block in the study blockchain, the blockchain and or the data contained therein may have been altered. If the recreated hash does match the hash of the second block, then the data and the blockchain are verified (at least for the analyzed blocks). At the end of the study, once all the secure analytical data files are received from the mass analysis instruments 102, the study blockchain forms a secured, verifiable representation of all the results in the study.

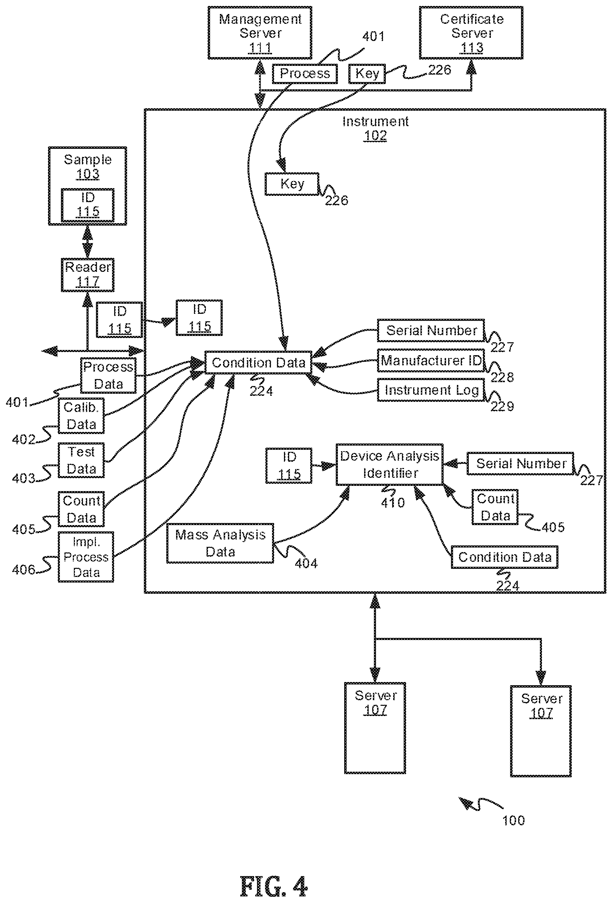

Attention is next directed to FIG. 4, FIG. 5, FIG. 6, and FIG. 7 which depict an example of the methods described above, such as method 320 depicted in FIG. 3B. Each of FIG. 4, FIG. 5, FIG. 6, and FIG. 7 depict a simplified version of the system 100. For instance, the components of the mass analysis instrument 102 are not depicted and the network 109 is not depicted, but the components of the mass analysis instrument 102 and the network 109 are nonetheless understood to be present.

Attention is first directed to FIG. 4, which depicts mass analysis instrument 102 receiving process data 401 and the key 226 and from the management server 111 and/or the certificate server 113, such as at the operation 322 of the method 320. While the memory 222 is not depicted in FIG. 4, it is understood that that the process data 401 and the key 226 are stored in the memory 222, for example at the operation 324 of the method 320. As depicted, the process data 401 is stored in the condition data 224.

FIG. 4 further depicts the mass analysis instrument 102 receiving the sample identifier 115 from a sample identifier reader 117, for example at operation 326 of the method 320. FIG. 4 further depicts the mass analysis instrument 102 receiving calibration data 402 and test data 403 which, as depicted, is stored in the condition data 224. The calibration data 402 and the test data 403 may be received prior to the sample identifier 115 and may be received as part of a calibration and/or test procedure of the mass analysis instrument 102 prior to analysis of the sample 103. FIG. 4 further depicts the mass analysis instrument 102 analyzing the sample 103, according to the condition data 224, for example, at operation 328 of the method 320, to generate mass analysis data 404, for example, at operation 330 of the method 320.

As depicted, the mass analysis instrument 102 may generate count data 405 (indicative of a run number and/or a cycle number for the analysis of the sample 103 that generated the mass analysis data 404) and implemented process data 406 indicative of an implemented process as the mass analysis instrument 102 implements the process based on the process data 401. For example, the process data 406 may include settings and/or instructions that the mass analysis instrument 102 is to implement to analyze the sample 103, and the implemented process data 406 represents the settings of the mass analysis instrument 402 as implemented. The implemented process data 406 may include machine-level characteristics of the mass analysis instrument 102 during performance of the tests on the sample. The operation 334 of method 320, discussed above, may be based on a comparison of the process data 401 with the implemented process data 406. As depicted, the count data 405 and the implemented process data 406 may also stored in the condition data 224. Hence, the condition data 224 represents a condition of the mass analysis instrument 102 when the sample 103 was analyzed. Furthermore, the calibration data 402, the test data 403, the process data 401, and the implemented process data 406 may also be stored in the instrument log data 229 which may represent a log of the operation of the mass analysis instrument 102.

Furthermore, as also depicted in FIG. 4, a device analysis identifier 410 may be generated from, for example, the sample identifier 115, the serial number 227, the count data 405, the condition data 224 and the mass analysis data 404. However, the device analysis identifier 410 may include any suitable combination of data. For example, when the count data 405 is incorporated into the condition data 224, the count data 405 may not be separately include in the device analysis identifier 410.

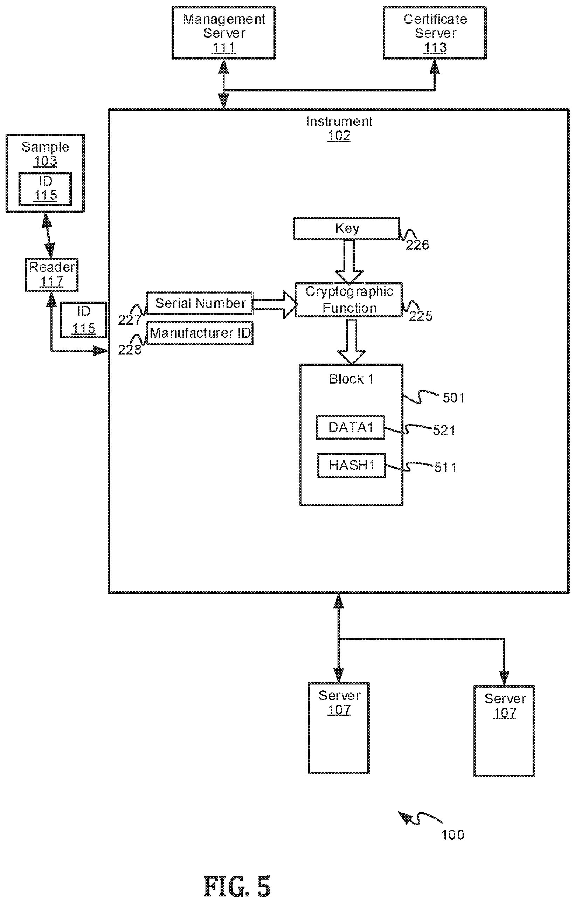

Attention is next directed to FIG. 5, which depicts a generation of a first block 501 (e.g., a genesis block) of a blockchain into which the mass analysis data 404 may be incorporated. While not all data stored at the mass analysis instrument 102 is depicted, it is nonetheless understood to be present. In particular, FIG. 5 depicts the key 226 being used as input to the cryptographic function 225 which produces the first block 501 including a hash 511 (also labelled "HASH1") of the serial number 227, and optionally the manufacturer identifier 228. The first block 501 further stores data 521 (also labelled "DATA1") which comprises all data used to produce the hash 511 (e.g., the serial number 227, and optionally the manufacturer identifier 228).

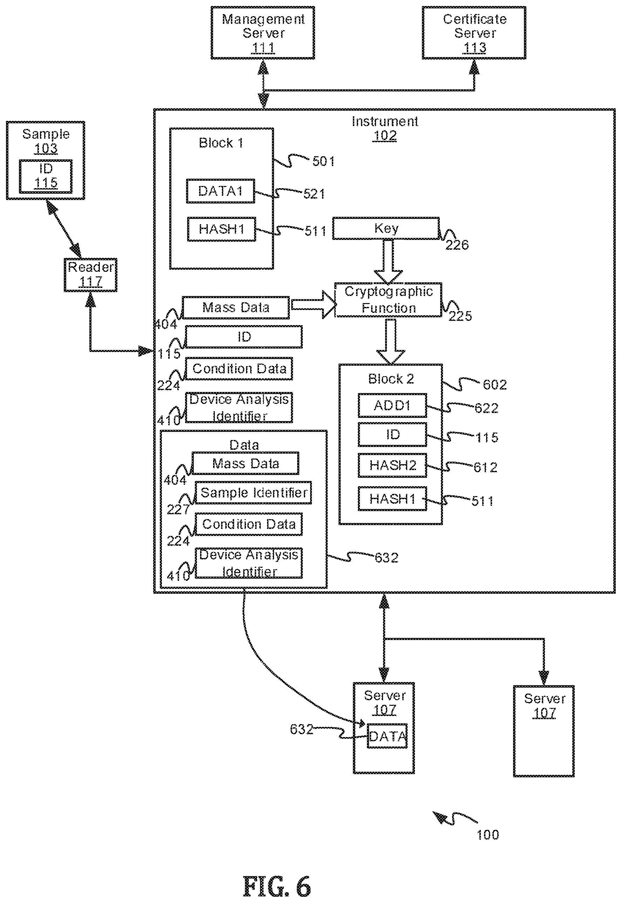

Attention is next directed to FIG. 6, which depicts a generation of a second block 602 of the blockchain into which the mass analysis data 404 is to be incorporated. In particular, FIG. 6 depicts the mass analysis instrument 102 performing, using at least the cryptographic function 225, the key 226, the mass analysis data 404, and the sample identifier 115, a cryptographic operation to generate (e.g., at operation 332 of the method 320) a cryptographic identifier, and specifically a hash 612 (also labelled "HASH2") of at least the mass analysis data 404 and the sample identifier 115. As depicted, however, the mass analysis instrument 102 also performs the cryptographic operation to generate the hash 612 of the combination of the mass analysis data 404, the sample identifier 115, the condition data 224 and the device analysis identifier 410. Indeed, in other examples, the hash 612 may comprise a hash of a combination of the mass analysis data 404 and one or more of: the sample identifier 115, the condition data 224, and the device analysis identifier 410.

FIG. 6 further also depicts generation of a verification file (e.g., at the operation 336 of the method 320). As depicted, the second block 602 includes: the hash 612 stored in association with the sample identifier 115, the hash 511 of the data in the first block 501, and a network address 622 ("ADD1") of the data used to generate the hash 612. For example, as depicted, the mass analysis data 404 and one or more of the sample identifier 115, the condition data 224, and the device analysis identifier 410 may be transmitted to a server 107 (e.g., at the operation 344 of the method 320) as data 632 and stored in an associated memory having the address 622. Alternatively or additionally, the data 632 may be stored in the second block 602 in place of the address 622. Alternatively or additionally, the data 632 may be stored in memory 222 of the mass analysis instrument 102 (e.g., at the operation 342 of method 320) and the address 622 may comprise a network address of the data 632 as stored in the memory 222.

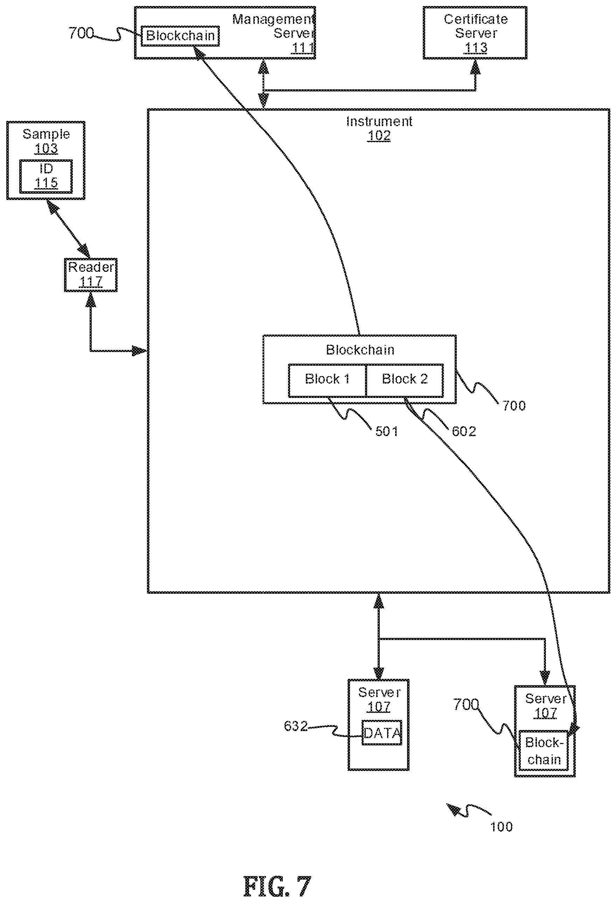

Attention is next directed to FIG. 7 which depicts a generation of a blockchain 700 from the blocks 501, 602, as well as transmission (e.g., at operation 340 of the method 320) of the blockchain 700 to the management server 111 and/or another of the servers 107 for storage in a respective memory. The blockchain 700 may also be stored (e.g., at operation 338 of the method 320) in the memory 222. Hence, the blockchain 700 may be stored in different locations for retrieval and verification against the data 632.

For example the verification may be performed by a device associated with an entity wishing to verify at least the mass analysis data 404 as well as one or more of: the sample identifier 115, the condition data 224, and the device analysis identifier 410. In particular, a computing device associated with a federal entity, such as the Food and Drug Administration (FDA) in the United States, and the like, may receive the mass analysis data 404 and/or the address 622, and wish to verify the mass analysis data 404 and one or more of: the sample identifier 115, the condition data 224 and the device analysis identifier 410. As such, the blockchain 700 may also be provided to such a computing device, as well as cryptographic function 225 and the key 226, and the computing device may verify the mass analysis data 404, and the like, against the blockchain 700. In other examples, the sample 103 may comprise saliva used to determine which may be tested for law enforcement purposes by the mass analysis instrument 102, and the blockchain 700 may be used by a legal entity as evidence.

In another example, when a plurality of samples are tested in an order, further blocks may be added to the blockchain 700, with each block (e.g., other than the first block 501) comprising a respective address of respective mass analysis data (as well as one or more of: a respective sample identifier, respective condition data 224 and respective device analysis identifier), the respective sample identifier, a respective hash of the respective data stored at the respective address, and a hash of the previous block.

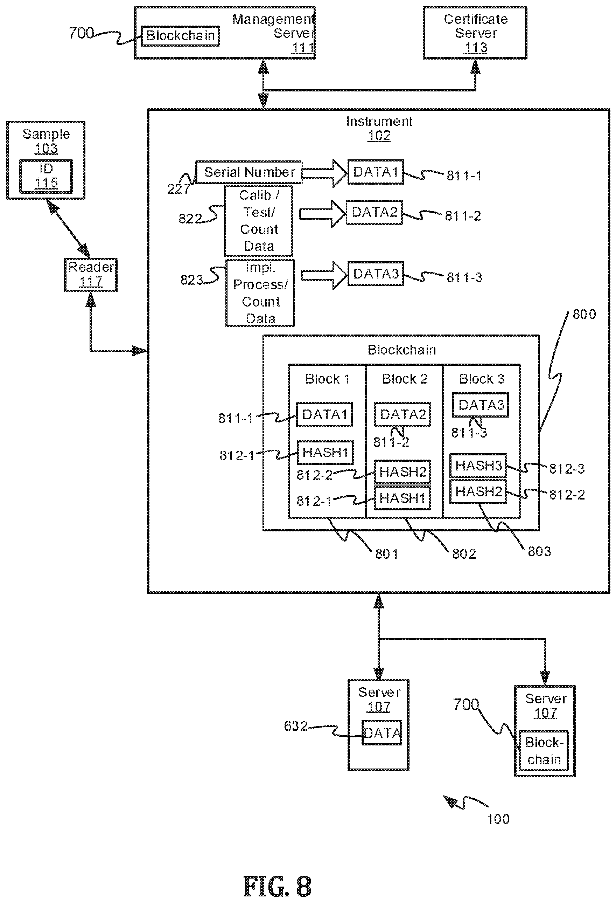

In yet further examples, such blockchaining techniques as described herein may be used to maintain a record of the operation of the mass analysis instrument 102. For example attention is next directed to FIG. 8 which depicts the mass analysis instrument 102 generating a blockchain 800 that includes a plurality of blocks 801, 802, 803; while three blocks are depicted, the blockchain may comprise as few as two blocks (e.g., the first block 801 and/or a genesis block, and the second block 802) or more than three blocks. In general, each block 802, 803 and higher represents a record of run and/or a cycle of the mass analysis instrument 102. Furthermore, each of the blocks 801, 802, 803 may be generated using the cryptographic function 225 and the key 226 as described above.

The first block 801 comprises data 811-1 (also labelled "DATA1") which, as depicted, may comprise the serial number 227 of the mass analysis instrument 102, as well as any other suitable data, similar as described above with respect to the first block 501. As depicted, the first block 801 further comprises a hash 812-1 (also labelled "HASH1") of the data 811-1.

The second block 802 comprises data 811-2 (also labelled "DATA2") and/or a network address of memory where the data 811-2 is stored. As depicted, the data 811-2 comprises a combination of calibration, test data and count data 822 (e.g., similar to the calibration data 402, the test data 403 and the count data 405) as well as any other suitable data. The count data in the data 822 may indicate a run and/or cycle of the mass analysis instrument 102 where the calibration data 402 and/or the test data 403 was acquired. As depicted, the second block 802 further comprises a hash 812-2 (also labelled "HASH2") of the data 811-2 and the hash 812-1 of the first block 801.

Similarly, the third block 803 comprises data 811-3 (also labelled "DATA3") and/or a network address of memory where the data 811-3 is stored. As depicted, the data 811-3 comprises a combination of implemented process data and count data 823 (e.g., similar to the implemented process data 406 and the count data 405) as well as any other suitable data, for example process data similar to the process data 401. As depicted, the third block 803 further comprises a hash 812-3 (also labelled "HASH3") of the data 811-3, and the hash 812-2 of the second block 802.

Hence, for each operational cycle of the mass analysis instrument 102, a block may be added to the blockchain 800 to maintain a record of the operation of the mass analysis instrument 102. Furthermore, the data stored in each block (and/or the data stored at an address in each block) may be customized based on operation at a given cycle. For example, as depicted, the second block 802 includes calibration and/or test data and the third block includes implemented process data.

While not depicted, the blockchain 800 may be stored at a remote storage device, such as one or more of the servers 107, 111, 113 and used to verify operation of the mass analysis instrument 102. For example, a computing device associated with a federal entity and/or a legal entity and the like, and/or by any other suitable computing device, may perform the verification operation.

As should be appreciated from the foregoing, provided herein, among other things, are systems and methods for verification of mass analysis data in which cryptographic and/or blockchaining techniques are used to generate a verification file or verification data used to verify mass analysis data and/or operation of a mass analysis instrument used to produce the mass analysis data. The verification file may be provided in a blockchain format such that a record of the conditions under which mass analysis data were produced may be easily verifiable and/or a record of operation of the mass analysis instrument 102 may be easily verifiable.

In this specification, elements may be described as "configured to" perform one or more functions or "configured for" such functions. In general, an element that is configured to perform or configured for performing a function is enabled to perform the function, or is suitable for performing the function, or is adapted to perform the function, or is operable to perform the function, or is otherwise capable of performing the function.

It is understood that for the purpose of this specification, language of "at least one of X, Y, and Z" and "one or more of X, Y and Z" can be construed as X only, Y only, Z only, or any combination of two or more items X, Y, and Z (e.g., XYZ, XY, YZ, XZ, and the like). Similar logic can be applied for two or more items in any occurrence of "at least one..." and "one or more..." language.

The terms "about", "substantially", "essentially", "approximately", and the like, are defined as being "close to", for example as understood by persons of skill in the art. In some examples, the terms are understood to be "within 10%," in other examples, "within 5%", in yet further examples, "within 1%", and in yet further examples "within 0.5%".

Persons skilled in the art will appreciate that in some examples, the functionality of devices and/or methods and/or processes described herein can be implemented using pre-programmed hardware or firmware elements (e.g., application specific integrated circuits (ASICs), electrically erasable programmable read-only memories (EEPROMs), etc.), or other related components. In other examples, the functionality of the devices and/or methods and/or processes described herein can be achieved using a computing apparatus that has access to a code memory (not shown) which stores computer-readable program code for operation of the computing apparatus. The computer-readable program code could be stored on a computer readable storage medium which is fixed, tangible and readable directly by these components, (e.g., removable diskette, CD-ROM, ROM, fixed disk, USB drive). Furthermore, it is appreciated that the computer-readable program can be stored as a computer program product comprising a computer usable medium. Further, a persistent storage device can comprise the computer readable program code. It is yet further appreciated that the computer-readable program code and/or computer usable medium can comprise a non-transitory computer-readable program code and/or non-transitory computer usable medium. Alternatively, the computer-readable program code could be stored remotely but transmittable to these components via a modem or other interface device connected to a network (including, without limitation, the Internet) over a transmission medium. The transmission medium can be either a non-mobile medium (e.g., optical and/or digital and/or analog communications lines) or a mobile medium (e.g., microwave, infrared, free-space optical or other transmission schemes) or a combination thereof.

Persons skilled in the art will appreciate that there are yet more alternative examples and modifications possible, and that the above examples are only illustrations of one or more examples. The scope, therefore, is only to be limited by the claims appended hereto.

## Claims

1. A method for generating verified mass analysis results for a sample, the method comprising:
acquiring process data for performing a mass analysis test on the sample;
performing, by a mass analysis instrument, the mass analysis test based on the acquired process data to generate mass analysis results for the sample;
recording machine-level characteristics for the mass analysis instrument during the mass analysis test;
generating verification data that includes at least a portion of the recorded machine-level characteristics;
generating a secure analytical data file that includes the verification data and at least a portion of the mass analysis results;
transmitting the secure analytical data file;
comparing the machine-level characteristics to the process data to determine whether the mass analysis test was performed in accordance with the process data; and
based on the comparison, generating a verification certificate, wherein the verification certificate is incorporated into the verification data.

2. The method of claim 1, further comprising logging at least one of calibration routine data, maintenance routine data, and cleaning routine data, wherein the verification data further includes the logged at least one of calibration routine data, maintenance routine data, and cleaning routine data.

3. The method of any one of claims 1-2, wherein the process data is acquired from a server managed by an entity requesting the mass analysis test;
optionally wherein the secure analytical data file is transmitted to the server.

4. The method of any one of claims 1-3, further comprising encrypting the secure analytical data file prior to transmission.

5. The method of any one of claims 1-4, wherein at least a portion of the secure analytical data file is stored as a block in a blockchain.

6. A mass analysis instrument comprising:
a communication unit;
a memory storing: condition data at least defining a process for analyzing the sample; a cryptographic function; and a key for input to the cryptographic function; and
a controller configured to:
receive a sample identifier of the sample;
control, according to the condition data, mass analysis instrument to analyze the sample according to mass to produce mass analysis data;
perform, using at least the cryptographic function, the key, and the mass analysis data, a cryptographic operation to generate a cryptographic identifier;
generate a verification file storing the cryptographic identifier in association with the sample identifier; and one or more of:
store, in the memory, the verification file; and
transmit, using the communication unit, the verification file for storage in a remote memory;
compare the process as stored in the condition data with an implemented process performed by the mass analysis instrument; and
based on the process and the implemented process matching, generate the verification file.

7. The mass analysis instrument of claim 6, wherein the condition data further comprises one or more of:
an analysis cycle count of the mass analysis instrument;
calibration data of the mass analysis instrument;
test data of the mass analysis instrument;
instrument log data of the mass analysis instrument;
a serial number; and
a manufacturer identifier.

8. The mass analysis instrument of any one of claims 6-7, wherein the verification file has a blockchain format, the verification file further comprises a respective cryptographic identifier of a previous verification file in a blockchain;
optionally wherein the controller is further configured to generate a first block in the blockchain using one or more of:
the sample identifier;
calibration data of the mass analysis instrument;
test data of the mass analysis instrument;
instrument log data of the mass analysis instrument;
a serial number; and
a manufacturer identifier.

9. The mass analysis instrument of any one of claims 6-8, wherein the cryptographic function comprises a hash function, the key comprises a hashing key, and the cryptographic identifier comprises a combination of the mass analysis data and the sample identifier generated using the hash function and the hash key.

10. The mass analysis instrument of any one of claims 6-9, wherein the controller is further configured to encrypt the verification file prior to one or more of storage and transmission.

11. The mass analysis instrument of any one of claims 6-10:
wherein the controller is further configured to: receive, via the communication unit, from a remote computing device, the key and process data defining the process; and store, in the memory, the key and the process data in the condition data; and/or
wherein the mass analysis instrument further comprises a sample identifier reader, and wherein the controller is further configured to determine the sample identifier by controlling the sample identifier reader to read the sample identifier from the sample.

12. A method for procuring and verifying mass analysis results, the method comprising:
generating a request for mass analysis tests of samples by a plurality of mass analysis instruments, wherein the request includes process data for performing the mass analysis tests;
transmitting the request to the plurality of mass analysis instruments;
receiving, from the plurality of mass analysis instruments, secure analytical data files, wherein the secure analytical data files include mass analysis results for the samples and verification data, wherein:
the verification data includes at least a portion of recorded machine-level characteristics for the mass analysis instrument during the mass analysis test; and
the mass analysis results are verified by comparing the machine-level characteristics to the process data to determine whether the mass analysis test was performed in accordance with the process data, and based on the comparison, generating a verification certificate, wherein the verification certificate is incorporated into the verification data; and
performing one or more verification operations on the received secure analytical data files.

13. The method of claim 12, wherein the process data includes at least one of settings and parameters for the mass analysis instrument.

14. The method of any one of claims 12-13, further comprising encrypting the request with a public key of one or more of the plurality of mass analysis instruments; and/or
wherein the one or more verification operations include decrypting the secure analytical data files with a private key.

15. The method of any one of claims 12-14, further comprising generating a first block in a study blockchain based on data in the request;
optionally wherein the first block includes data in the request and a first hash of the data in the request; and
optionally wherein at least a portion of the received secure analytical data files is provided within a second block of the study blockchain.

## Patentansprüche

1. Verfahren zum Erzeugen verifizierter Massenanalyseergebnisse für eine Probe, das Verfahren umfassend:
Erwerben von Prozessdaten zum Durchführen eines Massenanalysetests an der Probe;
Durchführen, durch ein Massenanalyseinstrument, des Massenanalysetests basierend auf den erworbenen Prozessdaten, um Massenanalyseergebnisse für die Probe zu erzeugen;
Aufzeichnen von Merkmalen auf Maschinenebene für das Massenanalyseinstrument während des Massenanalysetests;
Erzeugen von Verifizierungsdaten, die zumindest einen Teil der aufgezeichneten Merkmale auf Maschinenebene einschließen;
Erzeugen einer sicheren Analysedatendatei, die die Verifizierungsdaten und zumindest einen Teil der Massenanalyseergebnisse einschließt;
Übertragen der sicheren Analysedatendatei;
Vergleichen der Merkmale auf Maschinenebene mit den Prozessdaten, um zu bestimmen, ob der Massenanalysetest in Übereinstimmung mit den Prozessdaten durchgeführt wurde; und
basierend auf dem Vergleich, Erzeugen eines Verifizierungszertifikats, wobei das Verifizierungszertifikat in die Verifizierungsdaten aufgenommen wird.

2. Verfahren nach Anspruch 1, weiter umfassend Protokollieren von mindestens einem aus Kalibrierroutinedaten, Wartungsroutinedaten und Reinigungsroutinedaten, wobei die Verifizierungsdaten weiter das protokollierte mindestens eine aus Kalibrierroutinedaten, Wartungsroutinedaten und Reinigungsroutinedaten einschließen.

3. Verfahren nach einem der Ansprüche 1-2, wobei die Prozessdaten von einem Server erworben werden, der durch eine Entität verwaltet wird, die den Massenanalysetest anfordert;
wahlweise, wobei die sichere Analysedatendatei an den Server übertragen wird.

4. Verfahren nach einem der Ansprüche 1-3, weiter umfassend Verschlüsseln der sicheren Analysedatendatei vor der Übertragung.

5. Verfahren nach einem der Ansprüche 1-4, wobei zumindest ein Teil der sicheren Analysedatendatei als Block in einer Blockchain gespeichert wird.

6. Massenanalyseinstrument umfassend:
eine Kommunikationseinheit;
einen Speicher, der Folgendes speichert: Zustandsdaten, die zumindest einen Prozess zum Analysieren der Probe definieren; eine kryptografische Funktion; und einen Schlüssel zur Eingabe in die kryptografische Funktion; und
eine Steuereinheit, die ausgebildet ist, um:
eine Probenkennung der Probe zu empfangen;
das Massenanalyseinstrument, nach den Zustandsdaten, zu steuern, um die Probe nach Masse zu analysieren, um Massenanalysedaten zu erzeugen;
unter Verwendung von zumindest der kryptografischen Funktion, dem Schlüssel, und den Massenanalysedaten, eine kryptografische Operation durchzuführen, um einen kryptografischen Identifikator zu erzeugen;
eine Verifizierungsdatei zu erzeugen, die den kryptografischen Identifikator in Zuordnung zu der Probenkennung speichert;
und eines oder mehrere von:
die Verifizierungsdatei in dem Speicher zu speichern; und
unter Verwendung der Kommunikationseinheit die Verifizierungsdatei zur Speicherung in einem entfernten Speicher zu übertragen;
den Prozess, wie in den Zustandsdaten gespeichert, mit einem durch das Massenanalyseinstrument ausgeführten implementierten Prozess zu vergleichen; und
basierend auf dem Übereinstimmen des Prozesses und des implementierten Prozesses, die Verifizierungsdatei zu erzeugen.

7. Massenanalyseinstrument nach Anspruch 6, wobei die Zustandsdaten weiter eines oder mehrere von Folgendem umfasst:
eine Analysezyklusanzahl des Massenanalyseinstruments;
Kalibrierdaten des Massenanalyseinstruments;
Testdaten des Massenanalyseinstruments;
Instrumentprotokolldaten des Massenanalyseinstruments;
eine Seriennummer; und
einen Herstelleridentifikator.

8. Massenanalyseinstrument nach einem der Ansprüche 6-7, wobei die Verifizierungsdatei ein Blockchain-Format aufweist, die Verifizierungsdatei weiter einen jeweiligen kryptografischen Identifikator einer vorhergehenden Verifizierungsdatei in einer Blockchain umfasst;
wahlweise, wobei die Steuereinheit weiter ausgebildet ist, um einen ersten Block in der Blockchain unter Verwendung von einem oder mehreren von Folgendem zu erzeugen:
die Probenkennung;
Kalibrierdaten des Massenanalyseinstruments;
Testdaten des Massenanalyseinstruments;
Instrumentprotokolldaten des Massenanalyseinstruments;
eine Seriennummer; und
einen Herstelleridentifikator.

9. Massenanalyseinstrument nach einem der Ansprüche 6-8, wobei die kryptografische Funktion eine Hashfunktion umfasst, der Schlüssel einen Hash-Schlüssel umfasst, und der kryptografische Identifikator eine Kombination der Massenanalysedaten und der Probenkennung umfasst, die unter Verwendung der Hashfunktion und des Hash-Schlüssels erzeugt wird.

10. Massenanalyseinstrument nach einem der Ansprüche 6-9, wobei die Steuereinheit weiter ausgebildet ist, um die Verifizierungsdatei vor einem oder mehreren aus Speicherung und Übertragung zu verschlüsseln.

11. Massenanalyseinstrument nach einem der Ansprüche 6-10:
wobei die Steuereinheit weiter ausgebildet ist, um: über die Kommunikationseinheit von einer entfernten Rechenvorrichtung, den Schlüssel und Prozessdaten, die den Prozess definieren, zu empfangen; und den Schlüssel und die Prozessdaten in dem Speicher in den Zustandsdaten zu speichern; und/oder
wobei das Massenanalyseinstrument weiter einen Probenkennungsleser umfasst, und wobei die Steuereinheit weiter ausgebildet ist, um die Probenkennung zu bestimmen, indem sie den Probenkennungsleser steuert, um die Probenkennung von der Probe zu lesen.

12. Verfahren zum Beschaffen und Verifizieren von Massenanalyseergebnissen, das Verfahren umfassend:
Erzeugen einer Anforderung für Massenanalysetests von Proben durch eine Vielzahl von Massenanalyseinstrumenten, wobei die Anforderung Prozessdaten zum Durchführen der Massenanalysetests einschließt;
Übertragen der Anforderung an die Vielzahl von Massenanalyseinstrumenten;
Empfangen, von der Vielzahl von Massenanalyseinstrumenten, sicherer Analysedatendateien, wobei die sicheren Analysedatendateien Massenanalyseergebnisse für die Proben und Verifizierungsdaten einschließen, wobei:
die Verifizierungsdaten zumindest einen Teil aufgezeichneter Merkmale auf Maschinenebene für das Massenanalyseinstrument während des Massenanalysetests einschließen; und
die Massenanalyseergebnisse verifiziert werden, indem die Merkmale auf Maschinenebene mit den Prozessdaten verglichen werden, um zu bestimmen, ob der Massenanalysetest in Übereinstimmung mit den Prozessdaten durchgeführt wurde, und basierend auf dem Vergleich, ein Verifizierungszertifikat erzeugt wird, wobei das Verifizierungszertifikat in die Verifizierungsdaten aufgenommen wird; und
Durchführen einer oder mehrerer Verifizierungsoperationen an den empfangenen sicheren Analysedatendateien.

13. Verfahren nach Anspruch 12, wobei die Prozessdaten mindestens eines aus Einstellungen und Parametern für das Massenanalyseinstrument einschließen.

14. Verfahren nach einem der Ansprüche 12-13, weiter umfassend Verschlüsseln der Anforderung mit einem öffentlichen Schlüssel von einem oder mehreren der Vielzahl von Massenanalyseinstrumenten; und/oder
wobei die eine oder mehrere Verifizierungsoperationen das Entschlüsseln der sicheren Analysedatendateien mit einem privaten Schlüssel einschließen.

15. Verfahren nach einem der Ansprüche 12-14, weiter umfassend Erzeugen eines ersten Blocks in einer Studien-Blockchain basierend auf Daten in der Anforderung;
wahlweise, wobei der erste Block Daten in der Anforderung und einen ersten Hashwert der Daten in der Anforderung einschließt; und
wahlweise, wobei zumindest ein Teil der empfangenen sicheren Analysedatendateien in einem zweiten Block der Studien-Blockchain bereitgestellt wird.

## Revendications

1. Procédé de génération de résultats d'analyse de masse vérifiés pour un échantillon, le procédé comprenant :
l'acquisition de données de processus pour réaliser un test d'analyse de masse sur l'échantillon ;
la réalisation, par un instrument d'analyse de masse, du test d'analyse de masse sur la base des données de processus acquises afin de générer des résultats d'analyse de masse pour l'échantillon ;
l'enregistrement de caractéristiques de niveau machine pour l'instrument d'analyse de masse pendant le test d'analyse de masse ;
la génération de données de vérification qui incluent au moins une partie des caractéristiques de niveau machine enregistrées ;
la génération d'un fichier de données analytiques sécurisé qui inclut les données de vérification et au moins une partie des résultats d'analyse de masse ;
la transmission du fichier de données analytiques sécurisé ;
la comparaison des caractéristiques de niveau machine aux données de processus pour déterminer si le test d'analyse de masse a été réalisé conformément aux données de processus ; et,
sur la base de la comparaison, la génération d'un certificat de vérification, dans lequel le certificat de vérification est incorporé dans les données de vérification.

2. Procédé selon la revendication 1, comprenant en outre la consignation d'au moins une parmi des données de routine d'étalonnage, des données de routine de maintenance, et des données de routine de nettoyage, dans lequel les données de vérification incluent en outre l'au moins une parmi des données de routine d'étalonnage, des données de routine de maintenance, et des données de routine de nettoyage consignées.

3. Procédé selon l'une quelconque des revendications 1 à 2, dans lequel les données de processus sont acquises depuis un serveur géré par une entité demandant le test d'analyse de masse ;
facultativement dans lequel le fichier de données analytiques sécurisé est transmis au serveur.

4. Procédé selon l'une quelconque des revendications 1 à 3, comprenant en outre le chiffrement du fichier de données analytiques sécurisé préalablement à la transmission.

5. Procédé selon l'une quelconque des revendications 1 à 4, dans lequel au moins une partie du fichier de données analytiques sécurisé est stockée en tant que bloc dans une chaîne de blocs.

6. Instrument d'analyse de masse comprenant :
une unité de communication ;
une mémoire stockant : des données de condition définissant au moins un processus pour l'analyse de l'échantillon ; une fonction cryptographique ;
et une clé pour une entrée dans la fonction cryptographique ; et,
un dispositif de commande configuré pour :
recevoir un identifiant d'échantillon de l'échantillon ;
commander, selon les données de condition, un instrument d'analyse de masse pour analyser l'échantillon en fonction de la masse afin de produire des données d'analyse de masse ;
réaliser, en utilisant au moins la fonction cryptographique, la clé et les données d'analyse de masse, une opération cryptographique afin de générer un identifiant cryptographique ;
générer un fichier de vérification stockant l'identifiant cryptographique en association avec l'identifiant d'échantillon ;
et un ou plusieurs parmi :
stocker, dans la mémoire, le fichier de vérification ; et
transmettre, en utilisant l'unité de communication, le fichier de vérification pour le stockage dans une mémoire distante ;
comparer le processus tel que stocké dans les données de condition avec un processus mis en œuvre réalisé par l'instrument d'analyse de masse ; et
sur la base du processus et du processus mis en œuvre qui correspondent, générer le fichier de vérification.

7. Instrument d'analyse de masse selon la revendication 6, dans lequel les données de condition comprennent en outre un ou plusieurs parmi :
un comptage de cycles d'analyse de l'instrument d'analyse de masse ;
des données d'étalonnage de l'instrument d'analyse de masse ;
des données de test de l'instrument d'analyse de masse ;
des données de journal d'instrument de l'instrument d'analyse de masse ;
un numéro de série ; et,
un identifiant de fabricant.

8. Instrument d'analyse de masse selon l'une quelconque des revendications 6 à 7, dans lequel le fichier de vérification présente un format de chaîne de blocs, le fichier de vérification comprend en outre un identifiant cryptographique respectif d'un fichier de vérification précédent dans une chaîne de blocs ;
facultativement dans lequel le dispositif de commande est en outre configuré pour générer un premier bloc dans la chaîne de blocs en utilisant un ou plusieurs parmi :
l'identifiant d'échantillon ;
des données d'étalonnage de l'instrument d'analyse de masse ;
des données de test de l'instrument d'analyse de masse ;
des données de journal d'instrument de l'instrument d'analyse de masse ;
un numéro de série ; et,
un identifiant de fabricant.

9. Instrument d'analyse de masse selon l'une quelconque des revendications 6 à 8, dans lequel la fonction cryptographique comprend une fonction de hachage, la clé comprend une clé de hachage, et l'identifiant cryptographique comprend une combinaison des données d'analyse de masse et de l'identifiant d'échantillon générée en utilisant la fonction de hachage et la clé de hachage.

10. Instrument d'analyse de masse selon l'une quelconque des revendications 6 à 9, dans lequel le dispositif de commande est en outre configuré pour chiffrer le fichier de vérification préalablement à un ou plusieurs parmi le stockage et la transmission.

11. Instrument d'analyse de masse selon l'une quelconque des revendications 6 à 10 :
dans lequel le dispositif de commande est configuré en outre pour : recevoir, via l'unité de communication, à partir d'un dispositif informatique distant, la clé et des données de processus définissant le processus ; et stocker, dans la mémoire, la clé et les données de processus dans les données de condition ; et/ou
dans lequel l'instrument d'analyse de masse comprend en outre un lecteur d'identifiant d'échantillon, et dans lequel le dispositif de commande est en outre configuré pour déterminer l'identifiant d'échantillon en commandant le lecteur d'identifiant d'échantillon pour lire l'identifiant d'échantillon à partir de l'échantillon.

12. Procédé pour se procurer et vérifier des résultats d'analyse de masse, le procédé comprenant :
générer une requête pour des tests d'analyse de masse d'échantillons par une pluralité d'instruments d'analyse de masse, dans lequel la requête inclut des données de processus pour réaliser les tests d'analyse de masse ;
transmettre la requête à la pluralité d'instruments d'analyse de masse ;
recevoir, à partir de la pluralité d'instruments d'analyse de masse, des fichiers de données d'analyse sécurisés, dans lequel les fichiers de données d'analyse sécurisés incluent des résultats d'analyse de masse pour les échantillons et des données de vérification, dans lequel :
les données de vérification incluent au moins une partie de caractéristiques de niveau machine enregistrées pour l'instrument d'analyse de masse pendant le test d'analyse de masse ; et,
les résultats d'analyse de masse sont vérifiés en comparant les caractéristiques de niveau machine aux données de processus pour déterminer si le test d'analyse de masse a été réalisé conformément aux données de processus, et sur la base de la comparaison, générer un certificat de vérification, dans lequel le certificat de vérification est incorporé dans les données de vérification ; et
réaliser une ou plusieurs opérations de vérification sur les fichiers de données d'analyse sécurisés reçus.

13. Procédé selon la revendication 12, dans lequel les données de processus incluent au moins un parmi des réglages et des paramètres pour l'instrument d'analyse de masse.

14. Procédé selon l'une quelconque des revendications 12 et 13, comprenant en outre le chiffrement de la requête avec une clé publique d'un ou plusieurs parmi la pluralité d'instruments d'analyse de masse ; et/ou,
dans lequel les une ou plusieurs opérations de vérification incluent le déchiffrement des fichiers de données d'analyse sécurisés avec une clé privée.

15. Procédé selon l'une quelconque des revendications 12 à 14, comprenant en outre la génération d'un premier bloc dans une chaîne de blocs d'étude sur la base de données dans la requête ;
facultativement dans lequel le premier bloc inclut des données dans la requête et une première valeur de hachage des données dans la requête ; et,
facultativement dans lequel au moins une partie des fichiers de données analytiques sécurisés reçus est fournie dans un deuxième bloc de la chaîne de blocs d'étude.
